(19) Europäisches Patentamt
European Patent Office
Office européen des brevets

(11) EP 0 367 110 B1

(12) EUROPEAN PATENT SPECIFICATION

(45) Date of publication and mention of the grant of the patent:
**11.08.1999 Bulletin 1999/32**

(21) Application number: **89119910.1**

(22) Date of filing: **26.10.1989**

(51) Int Cl.$^{6}$: **C07D 495/22**, C07D 495/14, A61K 31/55 // (C07D495/22, 249:00, 243:00, 333:00, 207:00), (C07D495/14, 249:00, 243:00, 333:00)

(54) **1,4-diazepine derivative and its pharmaceutical use**

1,4-Diazepin-Derivate und deren pharmazeutische Verwendung

Dérivés de 1,4-diazépines et leur utilisation pharmaceutique

(84) Designated Contracting States:
**AT BE CH DE ES FR GB GR IT LI LU NL SE**

(30) Priority:
**31.10.1988 JP 27546088**
**24.11.1988 JP 29706888**
**16.12.1988 JP 31801688**
**28.12.1988 JP 33162288**

(43) Date of publication of application:
**09.05.1990 Bulletin 1990/19**

(60) Divisional application: **94101416.9 / 0 606 103**
**95111206.9 / 0 677 524**

(73) Proprietor: **Eisai Co., Ltd.**
**Tokyo (JP)**

(72) Inventors:
- **Okano, Kazuo**
  **Tsukuba-gun Ibaraki (JP)**
- **Miyazawa, Shuhei**
  **Toride-shi Ibaraki (JP)**
- **Clark, Richard Stephen John**
  **Tsukuba-shi Ibaraki (JP)**
- **Abe, Shinya**
  **Ushiku-shi Ibaraki (JP)**
- **Kawahara, Tetsuya**
  **Tsukuba-shi Ibaraki (JP)**
- **Shimomura, Naoyuki**
  **Tsukuba-shi Ibaraki (JP)**
- **Asano, Osamu**
  **Tsukuba-shi Ibaraki (JP)**
- **Yoshimura, Hiroyuki**
  **Tsukuba-shi Ibaraki (JP)**
- **Miyamoto, Mitsuaki**
  **Waltham, MA 02154 (US)**
- **Sakuma, Yoshinori**
  **Tsuchiura-shi Ibaraki (JP)**
- **Muramoto, Kenzo**
  **Tsukuba-shi Ibaraki (JP)**
- **Obaishi, Hiroshi**
  **Tsukuba-shi Ibaraki (JP)**
- **Harada, Koukichi**
  **Tsukuba-shi Ibaraki (JP)**
- **Tsunoda, Hajime**
  **Tsukuba-shi Ibaraki (JP)**
- **Katayama, Satoshi**
  **Tsukuba-shi Ibaraki (JP)**
- **Yamada, Kouji**
  **Ushiku-shi Ibaraki (JP)**
- **Souda, Shigeru**
  **Ushiku-shi Ibaraki (JP)**
- **Machida, Yoshimasa**
  **Tuskuba-shi Ibaraki (JP)**
- **Katayama, Kouichi**
  **Tuskuba-shi Ibaraki (JP)**
- **Yamatsu, Isao**
  **Ushiku-shi Ibaraki (JP)**

(74) Representative:
**Hansen, Bernd, Dr. Dipl.-Chem. et al**
**Hoffmann Eitle,**
**Patent- und Rechtsanwälte,**
**Postfach 81 04 20**
**81904 München (DE)**

(56) References cited:
**DE-A- 3 724 031**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The invention provides new 1,4-diazepine derivatives and pharmacologically acceptable salts thereof, a process for preparing it and the pharmaceutical use. The compounds and the salts have an excellent medical activity.
In recent years, a platelet activating factor (hereinafter referred to simply as PAF) has attracted much attention and its relationship with various diseases is now being clarified. Now, it has been assumed that PAF takes part in not only inflammation, but also DIC, endotoxin shock, asthma, ulcers in alimentary canal, hepatisis and the rejection at the time of organ transplantation. In addition, attention has been drawn to PAF as a mediator which is one of allergic reactions.
Under these circumstances, there have been made investigations on compounds having the anti-PAF activity. Among these compounds, a 1,4-diazepine compound having the anti-PAF action has been proposed, for example, in Japanese Laid-open Patent Application No. 63-33382. However, a satisfactory anti-PAF agent which is adapted, particularly, for allergies such as asthma has not been developed yet.
Accordingly, we have continued investigations and studies over a long term with respect to 1,4-diazepine derivatives which have not only an excellent PAF-inhibiting activity, but also a long activity.
DE-A-37 24 031 reveals tetracine compounds showing the following formulae Ia and Ib

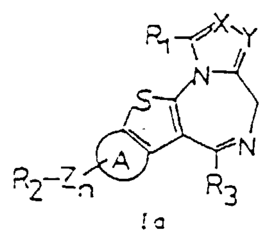

Ia

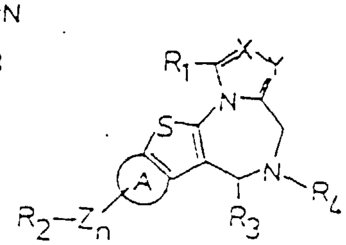

Ib

EP-0 194 416 A1 discloses thieno-triazolo-1,4-diazepino-2-amides with the following formula I

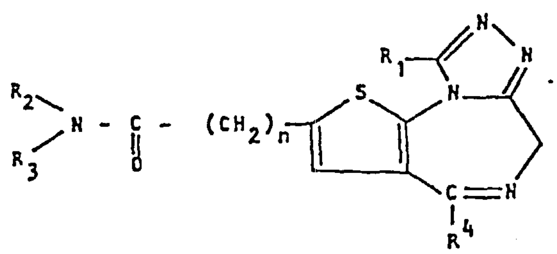

(I)

EP-0 230 942 discloses thieno-1,4-diazepin compounds of the general formulae

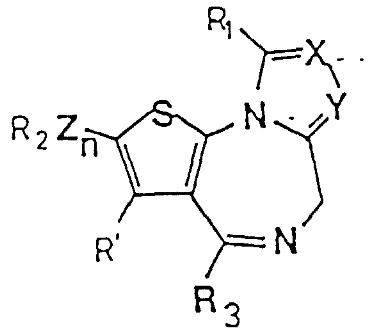

Ia

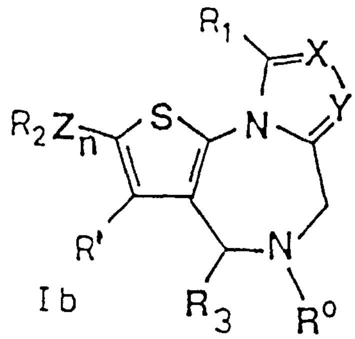

Ib

Finally, EP-A-268 242 discloses a thieno triazolodiazepine compound of the following structure type

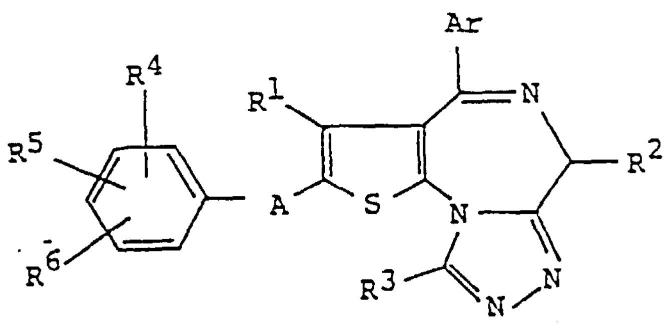

(I)

All of the above compounds are used for the treatment of diseases connected with PAF.

The invention provides triazolo-1,4-di-azepine compounds of the below given formula and pharmacologically acceptable salts thereof,

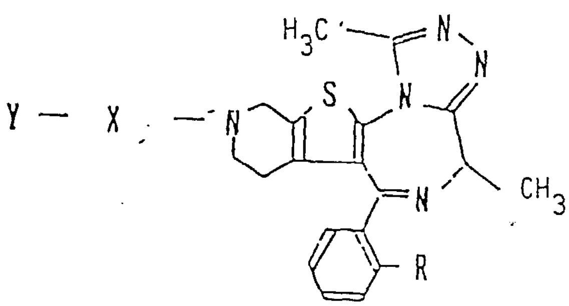

wherein R represents a hydrogen atom or a halogen atom, X represents a group of the formula

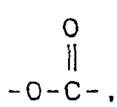

or

a group of the formula.

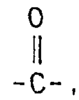

and Y represents

(1) a $C_3$-$C_7$ cycloalkyl group which may have a methyl group as a substituent(s), (2) a $C_3$-$C_7$ cycloalkyl-$C_1$-$C_6$ alkyl
(3) an alkynyl group having up to 6 C-atoms,
(4) a group of the formula,

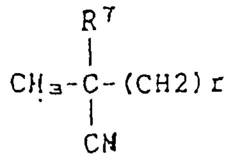

in which R7 is hydgrogen or methyl and r is zero, 1 or 2,
(5) a group of the formula, NC-$(CH_2)_P$-, (wherein p is an integer of from 1 to 6),
(6) a group of the formula, A-$(CH_2)_q$ wherein A represents a group selected from a pyridyl group, a pyranyl group and a morpholino group and q is an integer of from 0 to 6),
(7) an alkynyl group having up to 6 C-atoms wherein a phenyl group or a $C_3$-$C_7$ cycloalkyl group is joined to any carbon atom,
(8) a group of the formula,

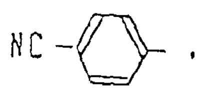

(9) a group of the formula,

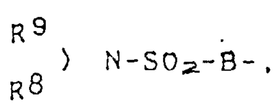

(wherein $R^8$ and $R^9$ are the same or different and represent a hydrogen atom, a $C_1$-$C_6$ alkyl group, a pyridylmethyl group or a $C_3$-$C_7$ cycloalkyl group or $R^8$ and $R^9$ may be joined along with a nitrogen atom to form a ring, and B represents a phenylene group or a $C_1$-$C_3$ alkylene group,
(10) a group of the formula,

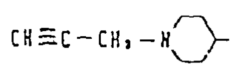

(11) a group of the formula,

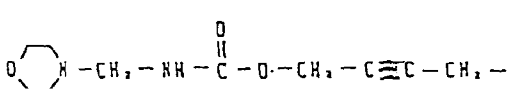

(12) a group of the formula,

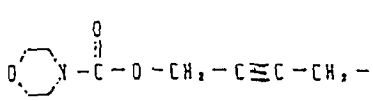

(13) a group of the formula,

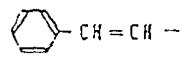

(14) a group of the formula,

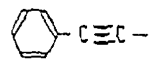

In the formula of the invention it is preferable that X is -CO-.

It is preferable that Y is one of (1) to (14), more preferably (4), (3), and (2). Most preferable examples for Y include a $C_3$-$C_7$ cycloalkyl such as cyclopropyl and HC=C-C(CH3)2-.

Preferable compounds have the formula in which R3 is chlorine:
and Y-X- is :

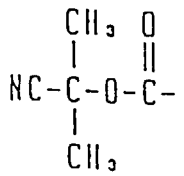 ,

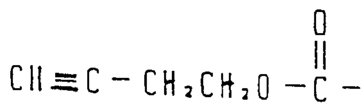 ;

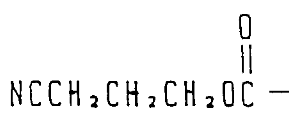

or

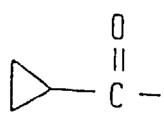

The invention provides the pharmacological use of the compound as defined above and its pharmaceutically acceptable salts. In the invention a pharmaceutical composition comprises a pharmacologically effective amount of the compound or a pharmaceutically acceptable salt thereof, defined above, and a pharmacologically acceptable carrier. A method for treating a disease against which anti-PAF activity is effective, which comprises administering a pharmacologically effective amount of the compound or pharmaceutically acceptable salts thereof as defined above. The disease is an allergic disease such as athma.

The 1,4-diazepine derivatives of the general formula (I) have good PAF-inhibiting efficacy and good persistency with high safety.

Accordingly, an object of the invention is to provide novel 1,4-diazepine derivatives or pharmacologically acceptable salts thereof which have good anti-PAF action. Another object of the invention is to provide a process for preparing the same. A further object pf the invention is to provide an agent comprising the same.

In the compounds (I) of the invention, R8 and R9 are a linear or branched $C_{1-6}$ alkyl and include, for example, a methyl group, an ethyl group, a propyl group, an isopropyl group, a butyl group, an isobutyl group, a sec-butyl group,

a tert-butyl group, a pentyl group (amyl group), an isopentyl group, a neopentyl group, a tert-pentyl group, a 1-methylbutyl group, a 2-methylbutyl group, a 1,2-dimethylpropyl group, a hexyl group, an isohexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group or the like. Of these, preferable groups include a methyl group, an ethyl group, a propyl group and an isopropyl group, of which the methyl group is most preferred.

The $C_{3-7}$ cycloalkyl group defined by Y is for example, cyclopropyl, cyclobutyl, cyclopentyl, cyclohexyl or cycloheptyl. Of these, cyclopropyl, cyclobutyl and cyclopentyl are most preferred. The cycloalkyl may have a substituent such as methyl.

The cycloalkylalkyl group is a group which is derived from the above-indicated cycloalkyl group. Typical examples include cyclopentylmethyl, cyclopropylmethyl, cyclohexylmethyl, and cyclohexylethyl groups.

The alkynyl group is a group which has up to 6 carbon atoms and a triple bond at any portion thereof. Typical alkynyl groups include, for example, $CH\equiv C-CH_2-$, $CH\equiv C-CH_2-CH_2-$, $CH\equiv C-CH_2-CH_2-CH_2-$, $CH\equiv C-CH_2-CH_2-CH_2-CH_2-$,

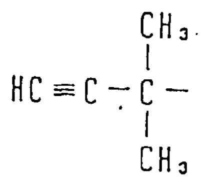

,

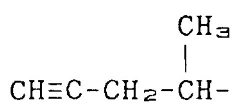

,

$CH_3-C\equiv C-CH_2-CH_2-$, and $CH_3-C\equiv C-CH_2-$.Of these, $CH\equiv C-CH_2-$, $CH\equiv C-CH_2-CH_2-$, $CH\equiv C-CH_2-CH_2-CH_2-$ and $CH\equiv C-CH_2-CH_2-CH_2-$ are most preferred.

In the formula (4),

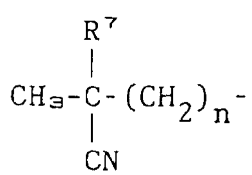

for Y, $R^7$ is most preferably a methyl group.

In the formula (5), p is from 1 to 6 preferably from 1 to 4.

In the formula (6), q is from 0 to 6.

In the formula (7), an alkynyl group having from 1 to 6 carbon atoms wherein a phenyl group or a cycloalkyl group is joined to any carbon atom of the group includes, for example, those groups of the following formulae

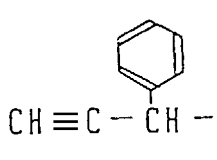

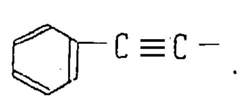

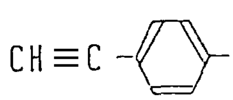

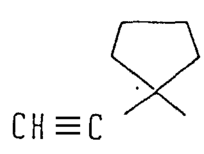

In (9), $R^8$ and $R^9$ may form a ring along with a nitrogen atom. Specific examples of the ring are shown below.

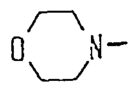 , 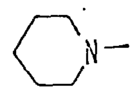 , 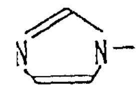 ,

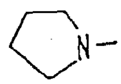 , 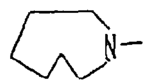 , 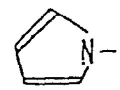

In the present invention, a first preferable group of compounds are those of the following chemical structural formula (A)

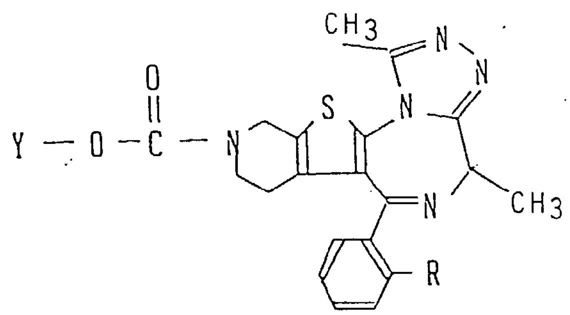

(A)

wherein R , and Y have, respectively, the same meanings as defined before where the most preferable group represented by Y is a group of the formula,

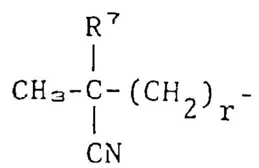 ,

in which R7 is hydrogen or methyl, r is zero, 1 or 2, a group of the formula $NC-(CH_2)_P-$ wherein p is an integer of from 1 to 6, or an alkynyl group. Most preferably, $R^7$ is a halogen atom such as a chlorine atom and (B)

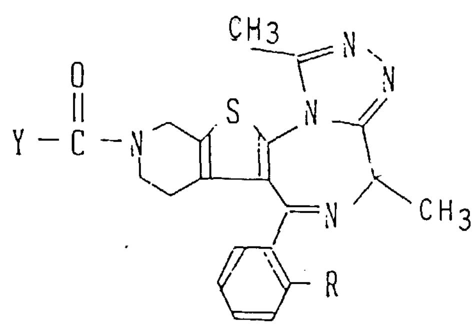

(B)

wherein R , and Y have, respectively, the same meanings as defined before where the most preferable group for Y is a cycloalkyl such as cyclopropyl, an alkynyl, a cycloalkylalkyl group, a group of the formula,

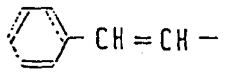

or a group represented by the formula, $NC-(CH_2)_P-$ wherein p is an integer of from 1 to 6.

In the compound (B), it is preferable that Y is cyclopropyl, and R is chlorine.

In the compounds of the present invention, the case where R is a halogen atom is most preferable. The most preferable halogen atom is a chlorine atom.

Y is most preferably a group of the formula,

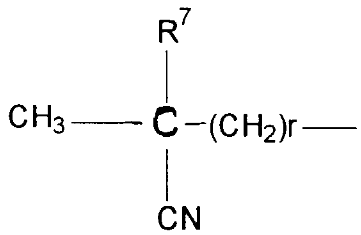

in which $R^7$ is hydrogen or methyl and r is zero, 1 or 2, a group of the formula, $NC-(CH_2)_P-$, wherein p is an integer of from 1 to 6, a $C_3$-$C_7$ cycloalkyl group, a $C_3$-$C_7$ cycloalkyl-$C_1$-$C_6$ alkyl group, or a group of the formula

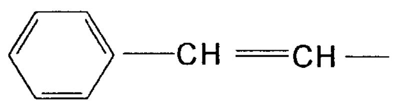

Especially, when Z is a group of the formula

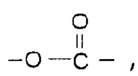

Y is most preferably an alkynyl group having up to 6 C-atoms such as, for example,
$CH\equiv C-CH_2-$, $CH\equiv C-CH_2-CH_2-$, $CH\equiv C-CH_2-CH_2-CH_2-$ or $CH\equiv C-CH_2-CH_2-CH_2-CH_2-$, A group of the formula,

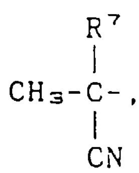

wherein $R^7$ has the same meaning as defined before, or a group of the formula, $NC-(CH_2)_P-$ (wherein p has the same meaning as defined before.

When X is a group of the formula,

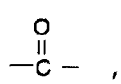

Y is most preferably a $C_3$-$C_7$ cycloalkyl group such as a cyclopropyl or cyclobutyl group, a $C_3$-$C_7$ cycloalkyl-$C_1$-$C_6$ alkyl group, an alkynyl group having up to 6 C-atoms, a group of the formula

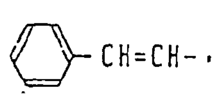

or a group of the formula, $NC-(CH_2)_p-$ wherein p is an integer of from 1 to 6.

These compounds wherein the methyl group is introduced into the diazepine ring exhibit unexpectedly better anti-PAF action than known 1,4-diazepine compounds as will be described hereinafter.

The pharmacologically acceptable salts used in the present invention are ordinarily employed innoxious salts such as, for example, inorganic salts such as hydrochlorides, hydrobromides, sulfates and phosphates, organic salts such as acetates, maleats, succinates and methanesulfonates, and salts of amino acids such as alginine, aspartic acid and glutamic acid.

The compounds of the invention have asymmetric carbon in the molecule and may take various steric isomers. In the practice of the invention, the individual isomers and mixtures thereof are all within the scope of the invention. The compounds of the present invention have an asymmetric carbon attached to methyl and therefore includes stereoisomers. The isomers can be obtained according to an usual process for preparation.

Moreover, some compounds may form hydrates, which are also within the scope of the invention.

The compounds of the invention are prepared by usual procedures, among which typical processes are described below.

Preparation Process 1

For the preparation of compounds of the formula (I) wherein X is of the formula (a),

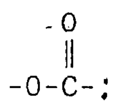

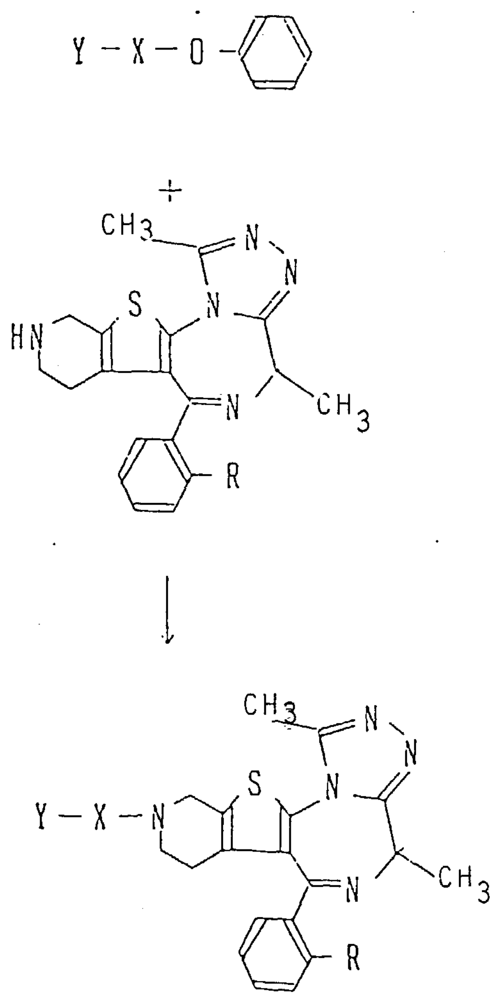

( II )

( III )

( I ' )

wherein Y, and R have, respectively, the same meaning as defined before.

The compound of the formula (II) and the compound of the formula (III) are subjected to condensation reaction to obtain the compound of the general formula (I') which is one of intended substances.

This reaction is performed by the usual manner in a solvent-free condition or in a solvent inert to the reaction and selected from chloroform, tetrahydrofuran, diethyl ether, acetone, benzene, toluene and dimethylformamide. The reaction temperature is generally from room temperature to approximately 150°C and most preferably from 100 to 130°C.

In the above reaction, the compound of the general formula (II) which is used as the starting material is prepared, for example, according to the following process.

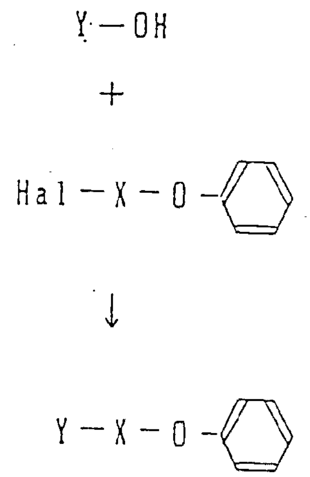

(IV)

(V)

(II)

wherein Y, and X have, respectively, the same meanings as defined before, and Hal represents a halogen atom.

In the above reaction, the compound of the general formula (IV) is subjected to condensation reaction with the halide of the general formula (V) to obtain the compound of the general formula (II).

The reaction should preferably be effected in the presence of bases including amines such as triethylamine and pyridine, alkali hydrides such as sodium hydride, and potassium hydride, and alkali hydroxides such as sodium hydroxide and, potassium hydroxide.

This reaction may be performed in the absence of solvent or in a solvent. Examples of the solvent include ethers such as tetrahydrofuran and dioxane, halogen-based compounds such as methylene chloride and chloroform, benzene compounds such as benzene, toluene or xylene, and compounds such as dimethylformamide and dimethylsulfoxide.

Preparation Process 2

For the preparation of compounds wherein X is of the formula,

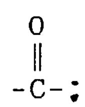

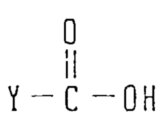

(VI)

or its reactive acid derivative

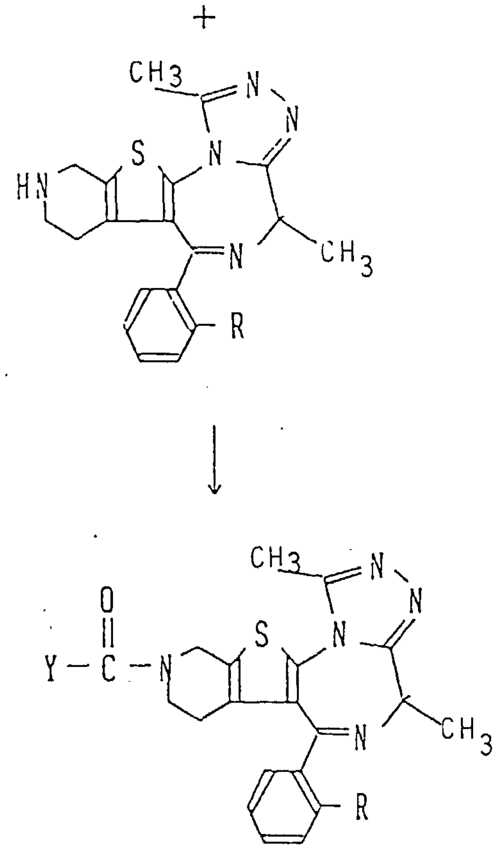

(III)

(I")

More particularly, the carboxylic acid of the general formula (VI) or its reactive derivative and the compound of the general formula (III) are subjected to condensation reaction to obtain the compound of the general formula (I") which is one of intended substances.

This condensation reaction is carried out by the usual manner. The reactive derivatives include: acid halides such as acid chlorides and acid bromides; acid azides; N-hydroxybenzotriazole; active esters such as N-hydroxysuccinimide; symmetric acid anhydrides; mixed acid anhydrides with alkali carbonates and p-toluenesulfonic acid.

This reaction is carried out by heating in a solvent-free condition or in a solvent not taking part in the reaction, e.g. benzene, toluene, xylene, tetrahydrofuran, chloroform, carbon tetrachloride or dimethylformamide, thereby causing, for example, dehalogenation reaction. Better results are obtained when the reaction is effected in the presence of inorganic salts such as sodium hydrogencarbonate, potassium carbonate, sodium carbonate and caustic soda or organic bases such as triethylamine, pyridine, pyrimidine and diethylaniline.

When free carboxylic acids are used, better results are obtained for the reaction in the presence of a condensing agent such as dicyclohexylcarbodiimide or 1,1'-carbonyldiimidazole.

The starting compound (III) used in the above Preparation Processes 1 and 2 can be prepared, for example, according to the following procedure.

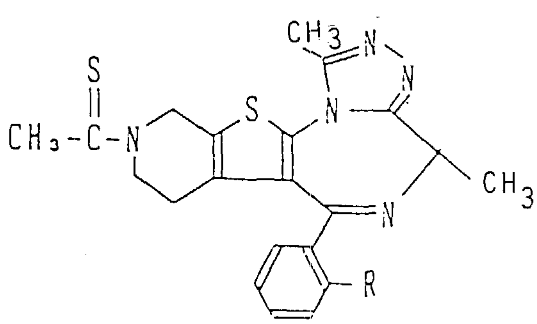

(IX)

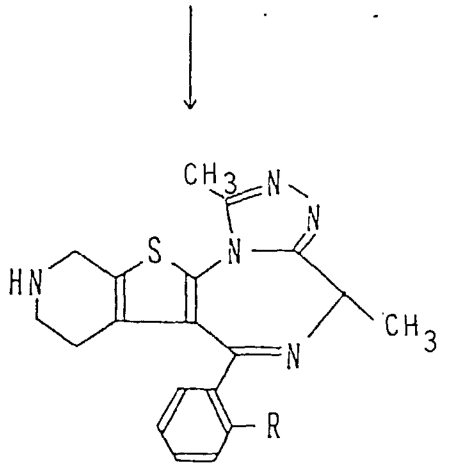

(III)

wherein R has the same meanings as defined before.

In the above reaction, the thioamide compound of the general formula (IX) is subjected to hydrolysis reaction to obtain the compound of the general formula (III).

The present reaction is carried out by the usual manner wherein the compound of the general formula (III) can be obtained by heating in the presence, for example, of sodium hydroxide, potassium hydroxide, sodium ethoxide, sodium methoxide, potassium ethoxide or potassium methoxide. For the reaction, there may be used a solvent such as, for example, an alcohol solvent such as methyl alcohol or ethyl alcohol, tetrahydrofuran, dimethoxyethane, or a hydrous solvent.

With the above starting compound (III) the preparation process can be more particularly described as follows.

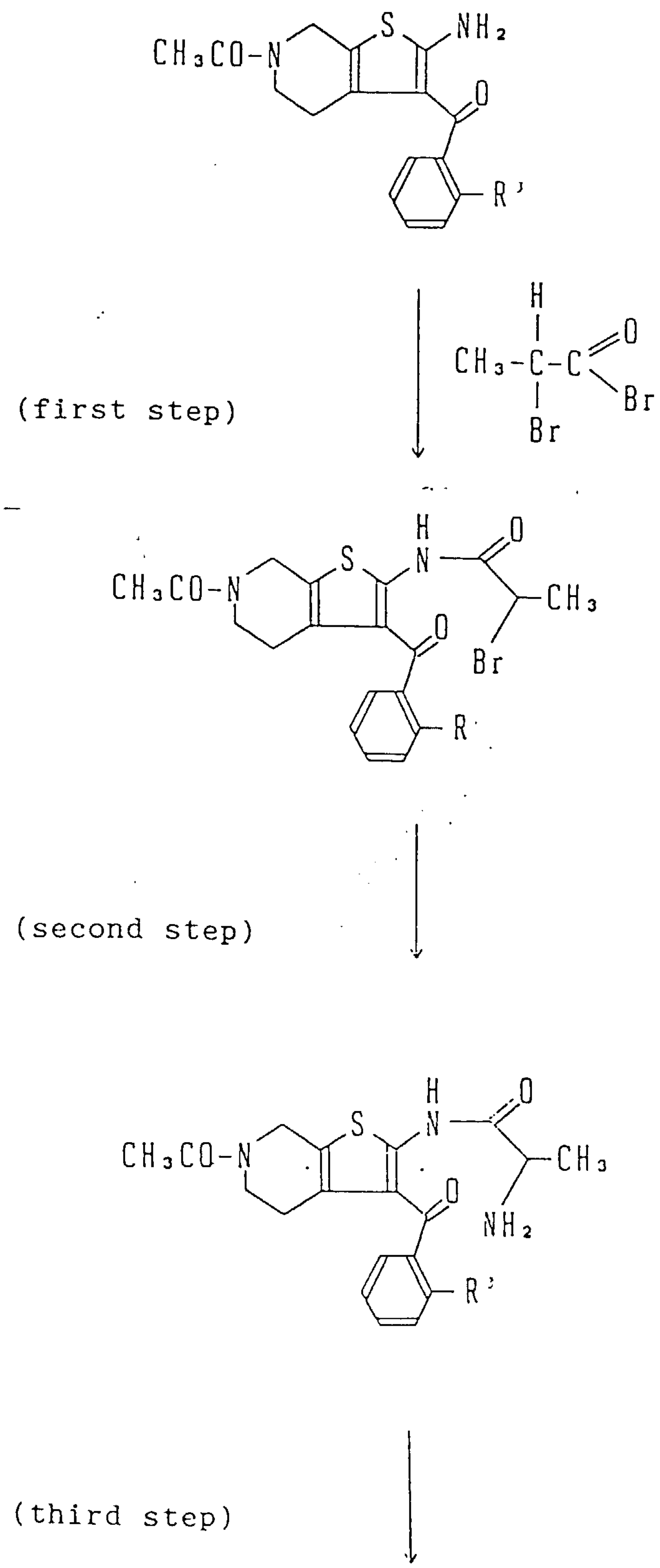


(X)

(XI)

(XII)

(XIII)

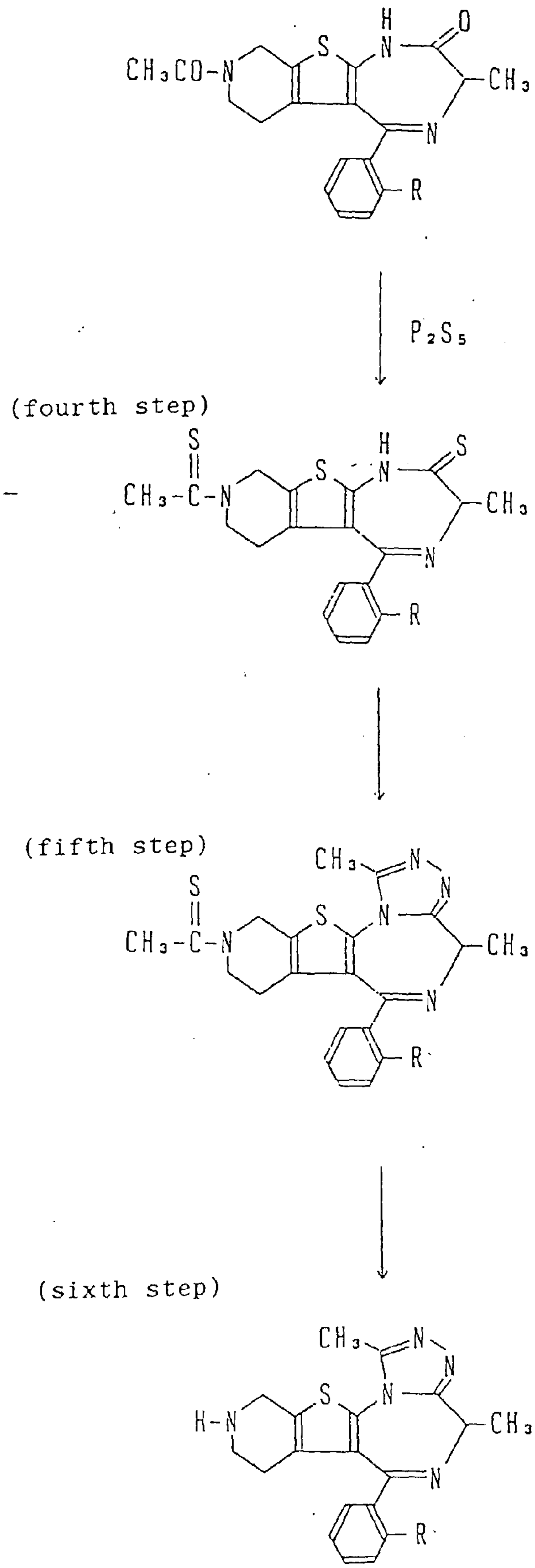


(XIV)

(XV)

(XVI)

(XVII)

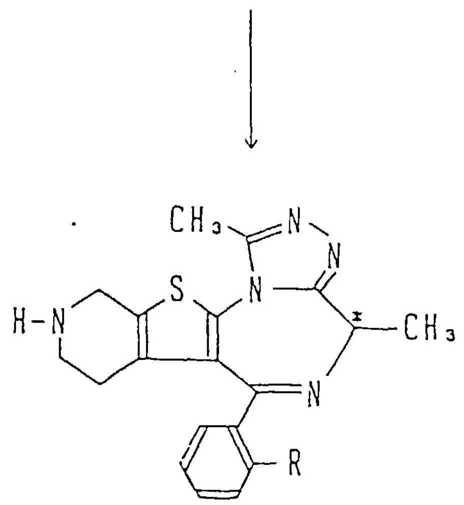

(XVIII)

wherein the mark "*" represents asymmetric carbon and (XVIII) represents the respective enanethiomer.

The respective steps indicated above are briefly illustrated in the following.

(First Step)

2-Bromopropionyl bromide of the formula (XI) is subjected to condensation reaction with the compound of the general formula (X) by the usual manner to obtain the compound of the general formula (XII).

This reaction is carried out in a two-phase system (under Schotten-Bauimann conditions) of an organic solvent such as, for example, toluene, benzene or xylene in the presence of either an alkali hydroxide such as sodium hydroxide or potassium hydroxide or a base such as sodium hydrogencarbonate or potassium hydrogencarbonate.

Alternatively, the reaction may be performed in the presence of a base including an amine such as triethylamine or pyridine, an alkali hydroxide such as sodium hydroxide or potassium hydroxide, or an alkali hydride such as sodium hydride or potassium hydride, in a solvent not taking part in the reaction such as, for example, dichloromethane, dichloroethane, tetrahydrofuran, toluene, benzene, xylene or dimethylformamide.

(Second Step)

In this step, ammonia gas is introduced into the compound of the general formula (XII) by the usual manner to obtain the compound (XIII).

This reaction should preferably be effected at low temperatures ranging, for example, from 30°C to 100°C.

The reaction is carried out in a solvent-free condition or by the use of an appropriate solvent which does not take part in the reaction and is selected from ethers such as tetrahydrofuran and dioxane, ethyl acetate, chloroform, methanol, ethanol, pyridine and dichloroethane.

(Third Step)

In this step, the compound of the general formula (XII) is subjected to dehydration reaction by the usual manner thereby causing cyclization to obtain the compound of the general formula (XIV).

One of the procedures is particularly described. The compound is dissolved in an appropriate solvent not taking part in the reaction such as, for example, benzene, toluene, xylene or pyridine, to which one equivalent of an acid catalyst such as acetic acid or silica gel. While removing the water produced as the reaction proceeds by the use of a dehydrator or by means of the Dean Stark apparatus, the reaction system is heated.

(Fourth Step)

This step is one wherein phosphorus pentasulfide is added to the compound of the general formula (XIV) for reaction to obtain the compound of the general formula (XV).

This reaction is carried out in a solvent such as pyridine, dimethoxyethane, diglymes, tetrahydrofuran, toluene, benzene or xylene. The reagent may be, aside from phosphorus pentasulfide, the Lauson reagent, (2,4-bis (4-methoxyphenyl9-1,3-dithia-2,4-diphosphetan-2,4-disulfide). In some cases, the reaction is carried out in the presence of a base such as sodium hydrogencarbonate.

(Fifth Step)

This step involves the reaction wherein acetohydrazide is reacted with the compound of the general formula (XV) to cause the cyclization reaction, thereby obtaining compound of the general formula (XVI).
This reaction is effected by heating acetohydrazide in a solvent which does not take part in the reaction such as, for example, dioxane, dimethoxyethane or diglymes or in a solvent-free condition. Alternatively, hydrazide hydrate is reacted in a solvent such as methanol or ethanol and the resultant hydrazide is reacted to ethyl ortho-acetate to obtain an intended product. Still alternatively, hydrazide may be reacted with acetyl chloride or acetic anhydride and the resultant product is dehydrated to obtain compound (XVI).

(Sixth Step)

This step is one wherein the compound of the general formula (XVI) is hydrolyzed by the usual manner to obtain the compound of the general formula (XVII).
This reaction proceeds according to known procedures. For instance, heating in the presence of potassium hydroxide, sodium hydroxide, sodium ethoxide, sodium methoxide, potassium ethoxide or potassium methoxide results in the compound of the general formula (XVII).
For the reaction, solvents may be used including alcohol solvents such as methyl alcohol or ethyl alcohol, tetrahydrofuran, dimethoxyethane or hydrous solvents.
A particular example of obtaining the compound of the general formula (XVII) through the above-described series of the reactions is illustrated in Preparatory Example appearing hereinafter wherein R is a chlorine atom.
The compound of the general formula (XVII) is a novel compound and is an important intermediate for obtaining final compounds having good anti-PAF activity.
The compounds of the present invention exhibit unexpectedly high anti-PAF activity when compared to known 1,4-diazepine compounds. In this sense, the compounds of the general formula (III), are very valuable as intermediates.
The intermediates have asymmetric carbon and thus optical isomers exist. In the practice of the invention, dℓ products may be resolved into optically active products, if desired.
The resolution may be performed at the stage of the compound of the general formula (XIII) wherein an optical resolving agent such as (+)-tartaric acid,, (+)-camphoric acid, (+)-dibenzoyltartaric acid, (+)-10-camphorsulfonic acid or (+)-mandelic acid may be used for the resolution. Alternatively, at the stage of the compound of the general formula (III) or (XVII), the resolution may be possible using an optical resolving agent such as dibenzoyl-D-tartaric acid or dibenzoyl-L-tartaric acid. Still alternatively, when using a column for optical isomer resolution such as, for example, a chiral polyamide silica gel HPLC (elute: tetrahydrofuran-hexane), the resolution at the stage of the compound of the general formula (XIII), (XVII) or (III) is possible.
The other compounds than those described for the processes for their preparations can be obtained in the same way, except for changing starting materials.
The effects of the invention are more particularlY described by way of the following experimental example.

Experimental Example

PAF Receptor Binding Assay to the Human Platelet (Method)

Platelets are obtained from healthy men according to the usual method and suspended at a concentration of $10^8$ platelets/460 µl in a binding buffer (10 mM phosphate-buffers saline (pH 7.0), with 0.1% (w/V) BSA and 0.9 mM $CaCl_2$). Platelets($10^8$) in 460 µl of the buffer were added to polypropylene tubes and preincubated with test compounds (20 µl), after vortexing, for 6 min at 37 °C. Subsequently, 20 µl of a binding buffer solution of $^3$H-PAF (final $^3$H-PAF concentration 0.6 - 1 nM) was added to the tubes, which were incubated for 6 minutes. The binding reaction was stopped by adding of 3 ml of an ice-cold washing solution (saline containing 0.1% (w/v) BSA). Platelets were isolated by vaccum filtration on glass filters (Whatman GF/C). After drying the glass filter, radioactivity on the glass filter was measured in scintilator with a liquid scintillation counter.
The inhibition percent is caluculated according to the following equation and the value of IC is determined by interpolation from the figure.

$$\text{Inhibition \%} = \frac{\text{(total binding) - (total binding with compound)}}{\text{(total binding ) - (non-specific binding total}}$$

binding : radioactivity of binding in the absence of cold PAF or test compounds
non-specific binding : radioactivity of binding in the presence of $10^{-5}$ M PAF

These results are shown in Table I.

non-specific binding : radioactivity (dpm) after the incubation with $10^{-5}$ M of cold PAF.

The results are shown in Table 1.

The mark "*" indicates asymmetric carbon and the marks "(+)" and "(-)" indicate specific rotation.
As shown in Table 1, it is obvious that these invented compounds have anti-PAF activity. Moreover, it has been found that the compounds possess more potent and long-acting anti-PAF activity, and show better safety properties than known compounds. Thus,- the present invention has a great merit.

Accordingly, the compounds will be effective for the therapy and prophylaxis of all diseases mediated by PAF.

Typical diseases for which the compounds are useful as a therapeutic and prophylactic agent include allergic diseases, asthma, thrombosis, cerebral apoplexy (cerebral hemorrhage, cerebral thrombosis), myocardial infarction, (angina pectoris), human disseminated intravascular coagulation syndrome (DIC), thrombophlebitis, glomerular hepatitis, anaphylactic shock and hemorrhagic shock. The invented compounds will be particularly useful as an anti-allergic agent and an anti-asthmatic agent.

When these compounds are administered as an anti-PAF agent, they may-be useful to orally dose in the form of a tablet, powder, granule, capsule or syrup.
Alternatively, they may be parenterally dosed as a suppositoty, injection, external remedy or drip. In the case of the invention, the compounds should preferably be used as an oral agent.

The dosage may depend on the type of disease, the degree of symptom and the age. When these compounds are orally administered, the doses of 0.001 - 10 mg/kg, preferably 0.01 - 0.5 mg/kg, will be beneficial.

For the preparations to be used as peroral and parenteral dose, they are made using ordinary, pharmaceutically acceptable additives. For the preparation of injections or drips, pH modifiers, buffer solutions, stabilizers and solubilizers are added to the principal ingredient, if necessary. The mixture can be freeze-dried, if necessary, to make injections for subcutaneous, intramuscular or intervenous or drip administrations.

Table 1

| Test compound | PAF receptor binding assay $IC_{50}$ (μM) |
|---|---|
| 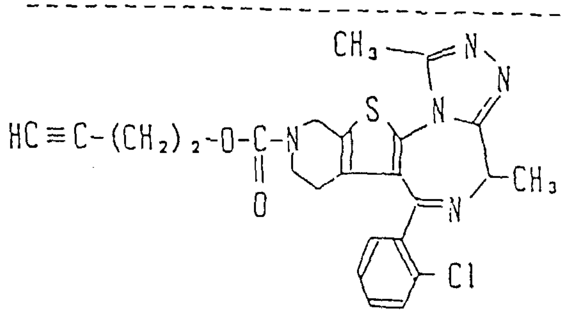 | 0.0015 |

Table 1 (Cont'd)

| | |
|---|---|
| 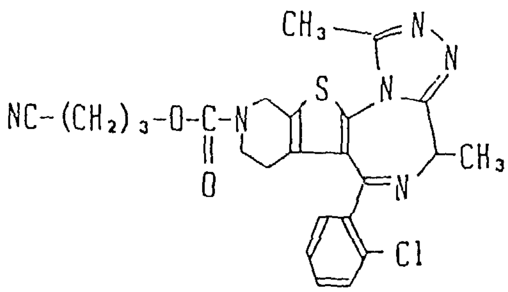 | 0.0044 |
| 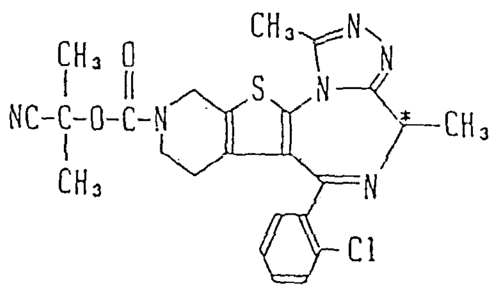 (−) | 0.5 |
| 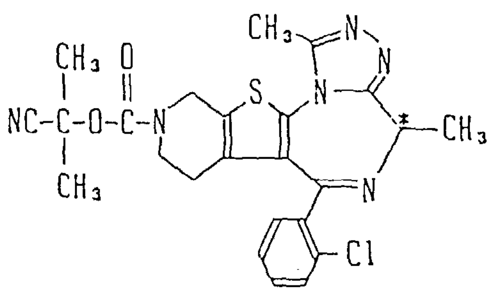 (+) | 0.0031 |
| 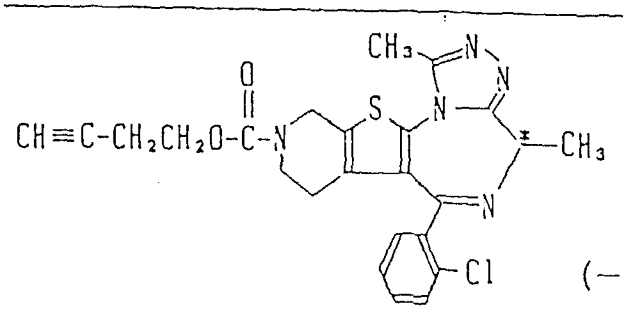 (−) | 0.082 |

Table 1 (Cont'd)

| | |
|---|---|
| 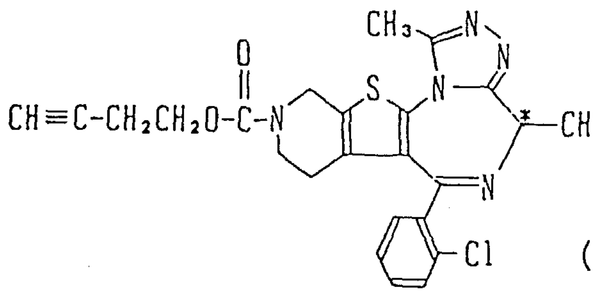 (+) | 0.00034 |
| 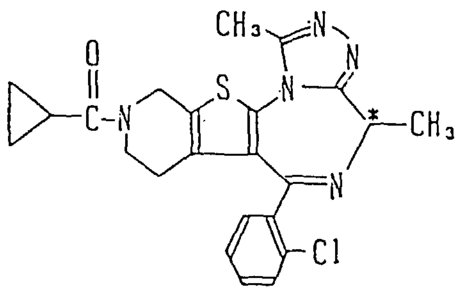 | 0.42 |
| 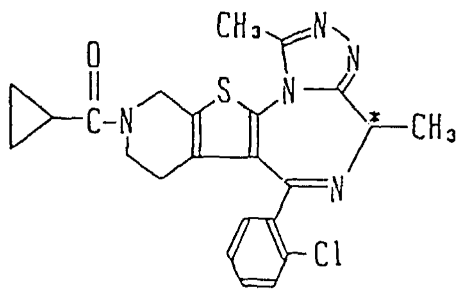 | 0.0028 |

[Examples]

Typical examples are as follows :
Examples 1 to 13 and Preparation Examples 1 to 33 are disclosed hereinafter.

It will be noted that the preparation of starting compounds or substances will be described as Preparatory Examples.

Example 1

6-(2-Chlorophenyl)-3-cyclopropanecarbonyl-8, 11-dimethyl-2,3,4,5-tetrahydro-8H-pyrido[4',3':4,5]thieno[3,2-f] [1,2,4] triazolo[4,3-a][1,4]diazepine

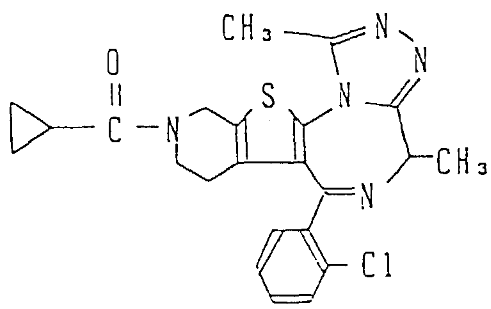

100 mg of 6-(2-chlorophenyl)-3-cyclopropanecarbonyl-11-methyl-2,3,4,5-tetrahydro-8H-pyrido[4',3':4,5] thieno[3,2-f][ 1,2,4]triazolo[4,3-a][1,4]diazepine was dissolved in 4 ml of N,N-dimethylformamide, to which 54 mg of sodium hydride (55%) and 0.5 ml of methyl bromide, followed by agitation for 1 hour at room temperature. The reaction was stopped by addition of water and the solution was neutralized with acetic acid. Subsequently, the solvent was distilled off under reduced pressure and the resultant residue was extracted with 20 ml of dichloromethane. The solution was dried with anhydrous magnesium sulfate, after which the solvent was removed, followed by purification with silica gel column chromatography (400 mesh, 10 g) to obtain the captioned product.

- $^1$H-NMR (90MHz, $CDCl_3$) δ :
  0.55 -1.15 (m, 4H), 1.45 - 2.5(m, 3H), 2.10(d, J=6.8 Hz,3H), 2.66(s, 3H), 2.8 - 4.8(m,3H), 4.26(q, J=6.8Hz, 1H), 4.8 - 5.2(m,1H), 7.05 - 7.65(m,4H)
- MS m/z(Pos. Fab): 412$(M+H)^+$

Example 2

3-(3-butynyloxycarbonyl)-6-(2-chlorophenyl)-8,11-dimethyl-2,3,4,5-tetrahydro-8H-pyrido[4',3':4,5]thieno[3,2-f][1,2,4] triazolo[4,3-a][1,4]diazepine

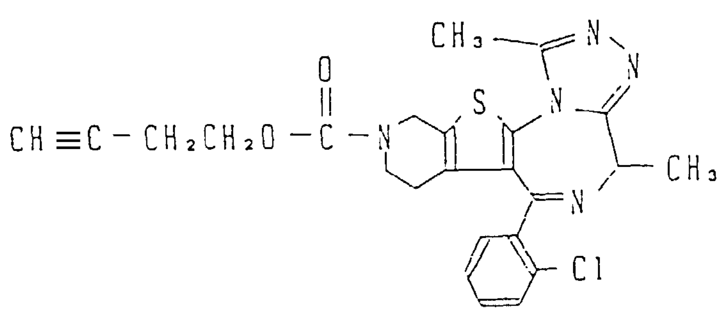

To a solution of 57 mg of 3-(3-butynyloxycarbonyl)-6-(2-chlorophenyl)-11-methyl-2,3,4,5-tetrahydro-8H-pyrido [4',3':4,5]thieno[3,2-f][ 1,2,4]triazolo[4,3-a][1,4]diazepine dissolved in dimethylformamide (2 ml) were added 28 mg of sodium hydride (55%) and 0.2 ml of methyl bromide, followed by agitation at room temperature for 1 hour.
Water was added to the solution in order to stop the reaction and the solution was neutralized with acetic acid. The solvent was distilled off under reduced pressure and the resultant residue was extracted with 10 ml of dichloromethylene and then with 20 ml of dichloromethylene. The solution was dried with magnesium sulfate and the solvent was removed, followed by purification with silica gel column chromatography (400 mesh, 10 g, elution solvent: methanol:dichloromethane = 1: 99), thereby obtaining 24 mg of the intended compound.

- NMR (90MHz, $CDCl_3$):
  7.4(5H,Ar), 4.9(1H,d,J=18Hz, N-$CH_2$[C-2]), 4.5(1H,d,J=18Hz,N-$CH_2$[C-2]), 4.2(1H,m,$C_8$-H), 4.1(2H,t, J=8Hz,O-$CH_2$), 2.7(3H,s), 2.5(2H,dt,J=1, 7Hz≡-$CH_2$), 2.1(3H,d,J=7Hz CHC$\underline{H}_3$), 3.0 - 2.0(5H,m)

Example 3

6-(2-Chlorophenyl)-8,11-dimethyl-3-(3-cyanopropoxycarbonyl)-2,3,4,5-tetrahydro-8H-pyrido[4',3':4,5]thieno[3, 2-f] [1,2,4]tri-azolo[4,3-a][1,4]diazepine

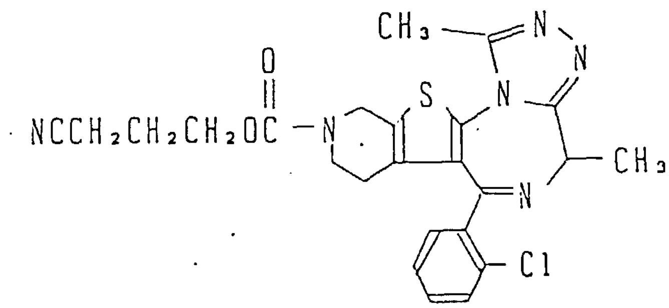

To a solution of 62 mg of 6-(2-chlorophenyl)-3-(3-cyanopropoxycarbonyl)-11-methyl-2,3,4,5-tetrahydro-8H-pyrido[4',3':4,5]thieno[3,2-f][1,2,4]triazolo-[4,3-a][1,4]diazepine dissolved in 2 ml of dimethylformamide at room temperature were added 34 mg of sodium hydride (55%) and 0.2 ml of methyl bromide, followed by agitation at room temperature for 1 hour. Water was added to the solution in order to stop the reaction and the solution was neutralized with acetic acid. The solvent was distilled off under reduced pressure and the resultant residue was extracted with 10 ml of dichloromethylene and then with 20 ml of dichloromethylene. The solution was dried with magnesium sulfate and the solvent was removed, followed by purification with silica gel column chromatography (400 mesh, 10 g, elution solvent:
methanol:dichloromethane = 1: 99), thereby obtaining 21 mg of the intended compound.

- NMR (90MHz, $CDCl_3$):
  7.4(5H,Ar), 4.9(1H,d,J=18Hz, N-$CH_2$[C-2]), 4.5(1H,d,J=18Hz,N-$CH_2$[C-2]), 4.2(1H,m,$C_8$-H), 4.1(2H,t, J=8Hz,0-$CH_2$), 2.7(3H,s), 2.4(3H,d,J=7Hz), 2.1(3H,d,J=7Hz CHC$\underline{H}_3$), 3.0 - 2.0(6H,m)

Example 4

6-(2-Chlorophenyl)-3-(1-cyano-1-methylethoxycarbonyl)-7,11-dimethyl-2,3,4,5-tetrahydro-8H-pyrido[4',3':4,5]thieno [3,2-f] [1,2,4]triazolo[4,3-a][1,4]diazepine

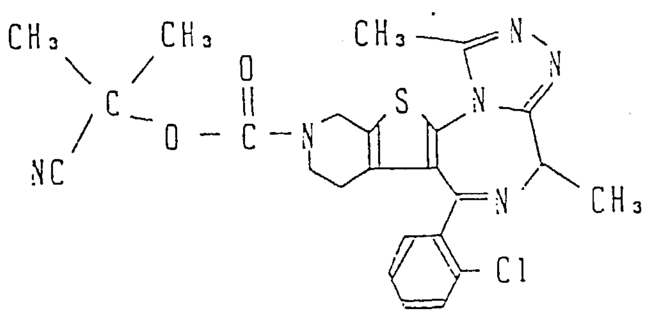

0.24 g of sodium hydride (60%) was added to a solution of 1.12 g of the compound obtained in Example 1 in N,N-dimethylformamide (3 ml) under ice-cooling conditions, followed by agitation for 30 minutes. Thereafter, 0.37 ml of methyl bromide was added to the solution and agitated under ice-cooling conditions for 30 minutes and then at room temperature for 1 hour. After completion of the reaction, water was added, followed by extraction with chloroform and drying with magnesium sulfate. The residue obtained after filtration and concentration was subjected to purification with silica gel column chromatography (elution solvent: chloroform:methanol = 99:1), thereby obtaining 0.19 g of the intended compound.

- $^1$H-NMR (90MHz, $CDCl_3$) δ :
  1.76(s,6H), 1.80 - 2.20(m,2H), 2.10(d,3H), 2.66(s,3H), 3.0 - 3.9(m,2H), 4.24(q,IH), 4.3 - 4.9(m,2H), 7.35(m, 4H),
- FABMS[M+H$^+$] m/z: 495

Examples 16 to 71

In the same manner as in the foregoing example, the following compounds were prepared.

Preparatory Example 1

<u>6-Acetyl-2-(2-bromopropionylamino)-3-(2-chlorobenzoyl)-4,5,6,7-tetrahydro-thieno[2,3-C]pyridine</u>

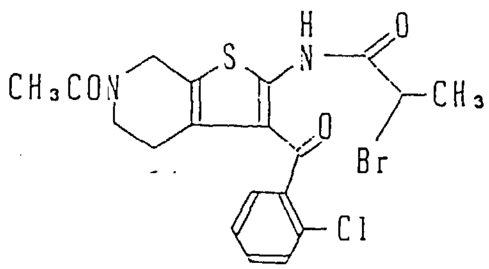

13.3 g of toluene and 3.66 liters of water were added to 600 g of 2-amino-3-(2-chlorobenzoyl)-6-acetyl-4,5,6,7-tetrahydro-thienq[2,3-C]pyridine, to which 301 g of sodium hydrogencarbonate was further added. While heating to 60°C, 301 ml of 2-bromopropionyl bromide was dropped into the solution. Further, 170 g of sodium hydrogencarbonate and 170 ml of 2-bromopropionyl bromide were added in order to complete the reaction. After cooling down to room temperature, 500 g of sodium hydrogencarbonate was added, after which the organic phase was separated. The aqueous phase was extracted twice with ethyl acetate, followed by combination with the organic phase, washing with water and drying with anhydrous magnesium sulfate. The solvent was distilled off under reduced pressure and the resultant solid matter was washed with ether to obtain 800 g of the intended product.

- $^1$H-NMR(90 MHz, $CDCl_3$) δ :
  1.7 - 2.4(m,2H), 1.99(d, J=7.2Hz, 3H), 2.06 and 2.12(each S, total 3H), 3.25 - 3.7(m,2H), 4.41(q, J=7.2Hz, 1H), 4.4 - 4.8(m,2H), 7.0 - 7.5(m,4H)

Preparatory Example 2

6-Acetyl-2-(2-aminopropionylamino)-3-(2-chlorobenzoyl)-4,5,6,7-tetrahydro-thieno[2,3-C]pyridine

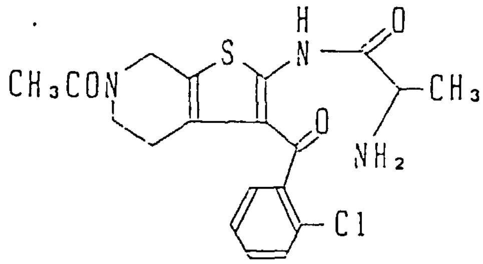

(process A)

841 g of 6-acetyl-2-(2-bromopropionylamino)-3-(2-chlorobenzoyl)-4,5,6,7-tetrahydro-thieno[2,3-C]pyrid ine was dissolved in 0.72 liters of dichloroethane and 1.08 liters of ethyl acetate, into which ammonia gas was introduced at -10°C. The mixture was subjected to reaction in an autoclave at 100°C for 1 hour. After completion of the reaction, excess ammonia gas was removed and the reaction solution was poured into 3N HCl under ice-cooling conditions. After extraction with ethyl acetate, the aqueous phase was neutralized with a saturated sodium carbonate aqueous solution, followed by repeating extraction with chloroform. The resultant organic phase was washed with a saturated saline solution, followed by drying with anhydrous magnesium sulfate and removal of the solvent by distillation under reduced pressure to obtain 636.8 g of the intended compound.

- $^1$H-NMR(90 MHz, $CDCl_3$) δ :
  1.48(d, J=6.8Hz; 3H), 1.6 - 2.3(m,4H), 2.07 and 2.12(each S, total 3H), 3.25 - 4.0(m,3H), 4.35 - 4.75(m,2H), 7.0 - 7.6(m,4H)

(process B)

Ten grams of 2-amino-3-(2-chlorobenzoyl)-6-acetyl-4,5,6,7-tetrahydro-thieno(2,3-C)pyridine was dissolved in 150 ml of chloroform at the room temperature. 17 g of alanyl chloride hydrochloride was added little by little to the solution over a period of 1 hour at the room temperature, while agitated. After the reaction finished, 150 ml of water was added to the mixture. Agitation was conducted for 30 minutes. The aqueous phase was taken out. The phase in chloroform was treated with 150 ml of water for extraction. The two aqueous phases were collected into one. It was washed with chloroform. The aqueous phase was neutralized with sodium bicarbonate and extacted with chloroform. The resultant phase was treated with a reduced pressure distillation to remove out the solvent and obtain 10.1 grams of the intended compound in the form of yellow powder.

Preparatory Example 3

8-Acetyl-5-(2-chlorophenyl)-3-methyl-6,7,8,9-tetrahydro-1H,3H-pyrido[4',3':4,5]thieno[3,2-f][1,4] diazepin-2-one

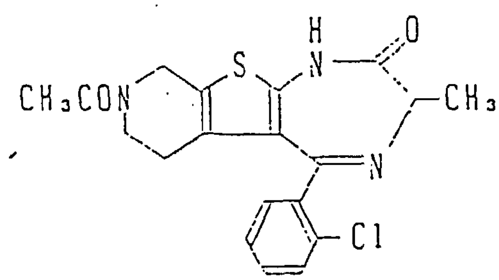

636.8 g of 6-acetyl-2-(2-aminopropionylamino)-3-(2-chlorobenzoyl)-4,5,6,7-tetrahydro-thieno[2,3-C]pyridine was dissolved in 2.3 liters of toluene, 637 ml of pyridine and 94.3 ml of acetic acid and refluxed over day and night while removing water from the reaction system. After removal of the reaction solution by distillation, benzene was added, followed by cooling and filtering the resultant crystals to obtain 300 g of the intended product.

- $^1H$-NMR(90 MHz, $CDCl_3$) δ :
  1.3 - 2.6(m,2H), 1.76(d,J=6.8Hz,3H), 2.06 and 2.12(each S, total 3H), 2.8 - 4.1(m,2H), 3.87(q,J=6.8Hz,IH), 4.1 - 5.1(m,2H), 7.1 - 7.5(m,4H), 9.0 - 9.5(bs,IH)

Preparatory Example 4

3-Methyl-5-(2-chlorophenyl)-8-thioacetyl-6,7,8,9-tetrahydro-1H,3H-pyrido[4',3':4,5]thieno[3,2-f][1,4] diazepin-2-thione

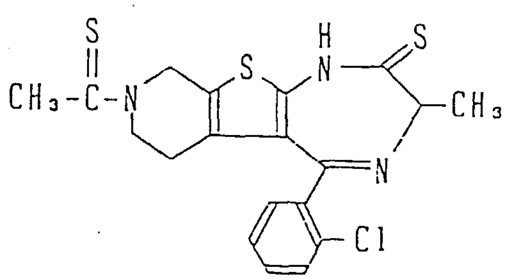

288 g of 3-methyl-5-(2-chlorophenyl)-8-acetyl-6,7,8,9-tetrahydro-1H,3H-pyrido[4',3':4,5]thieno[3,2-f][1,4] azepin-2-one was dissolved in 3 liters of dimethoxyethane, to which 186 g of sodium hydrogencarbonate and 364 g of phosphorus pentasulfide were added, followed by heating under reflux for 3 hours. The reaction solution was filtered through Celite, after which the solvent was once distilled off under reduced pressure. Methanol and dichloromethane were added to the resultant residue in small amounts for adsorption on silica gel, followed by drying and purification with dry column chromatography (elution solvent:
dichloromethane:methanol = 98:2), thereby obtaining 300 g of the intended compound.

Preparatory Example 5

6-(2-Chlorophenyl)-3-thioacetyl-8,11-dimethyl-2,3,4,5-tetrahydro-8H-pyrido[4',3':4,5]thieno[3,2-f][1,2,4] triazolo[4,3-a][1,4]diazepine

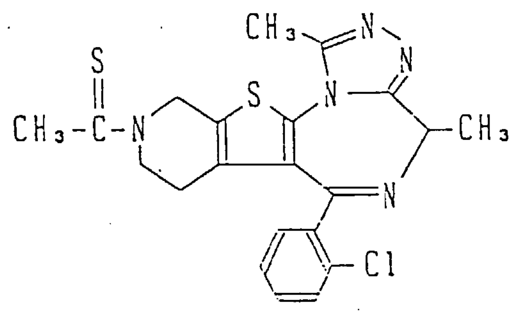

4.81 g of 3-methyl-5-(2-chlorophenyl-8-thioacetyl-6,7,8,9-tetrahydro-IH,3H-pyrido[4',3':4,5]thieno[3,2-f][1,4] diazepin-2-thione was dissolved in 70 ml of dioxane, to which 660 mg of acetohydrazide was added, followed by heating at 100°C. After cooling, the mixture was concentrated under reduced pressure and the resultant residue was purified by column chromatography (elution solvent: dichloromethane:methanol = 98:2), thereby obtaining 750 g of the intended compound.

Preparatory Example 6

6-(2-Chlorophenyl)-8,11-dimethyl-2,3,4,5-tetrahydro-8H-pyrido[4',3':4,5]thieno[3,2-f][1,2,4] triazolo[4,3-a][1,4] diazepine

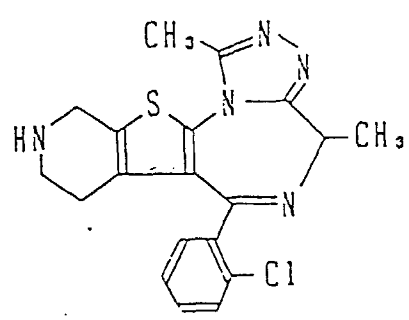

281 g of 6-(2-chlorophenyl)-8,11-dimethyl-3-thioacetyl-2,3,4,5-tetrahydro-8H-pyrido[4',3':4,5]thieno[3,2-f] [1,2,4] triazolo[4,3-a][1,4]diazepine was dissolved in 1 liter of methanol, to which 0.81 liters of 4N sodium hydroxide were added, followed by heating under reflux. After cooling, the reaction solution was salted out and extracted with chloroform, followed by removal of the solvent by distillation under reduced pressure. The resultant residue was purified by silica gel column chromatography (elution solvent: dichloromethane:methanol = 95:5), thereby obtaining 142-g of the intended compound.

- $^1$H-NMR(90 MHz, $CDCl_3$) $\delta$ :
  1.1 - 2.3(m,3H), 2.10(d,J=6.8Hz,3H), 2.45 - 3.3(m,2H), 2.66(s,3H), 3.85 - 4.1(m,2H), 4.26(q,J=6.8Hz,1H), 7.1 - 7.6(m,4H)
- MS m/z(Pos. Fab): 384(M+H)$^+$

Preparatory Example 7

(-)-6-(2-Chlorophenyl)-8,11-dimethyl-2,3,4,5-tetrahydro-8H-pyrido[4',3':4,5]thieno[3,2-f][1,2,4] triazolo[4,3-a][1,4] diazepine

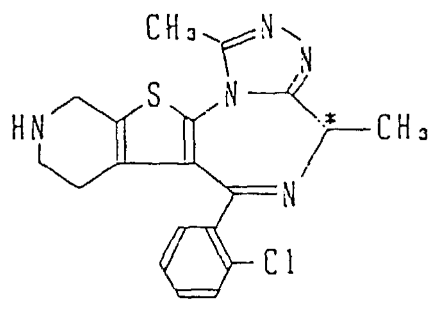

86 g of (±)-6-(2-chlorophenyl)-8,11-dimethyl-2,3,4,5-tetrahydro-8H-pyrido[4',3':4,5]thieno[3,2-f][1,2,4]triazolo [4,3-a][1,4]diazepine and 45.86 g of dibenzoyl-D-tartarate were dissolved under heating conditions in 980 ml of ethanol and 365 ml of water, and allowed to stand at room temperature. The resultant crystals were collected by filtration and washed with ether, followed by rendering free by means of a diluted sodium hydrogencarbonate aqueous solution and extraction twice with dichloromethane. The resultant organic phase was washed with a saturated saline solution and dried with anhydrous magnesium sulfate, followed by removal of the solvent by distillation under reduced pressure to obtain 11.0 g of the intended compound.

Further, the filtrate from which the tartarate had been once collected by filtration was subjected to a similar procedure set forth above, thereby obtaining 11.3 g of the intended compound.

- $[\alpha]_D^{26}$ -23.5° (C=1, EtOH)

Preparatory Example 8

(+)-6-(2-Chlorophenyl)-3-(1-cyano-1-methylethoxycarbonyl)-8,11-dimethyl-2,3,4,5-tetrahydro-8H-pyrido[4',3':4,5] thieno[3,2-f][1,2,4] triazolo[4,3-a][1,4]diazepine

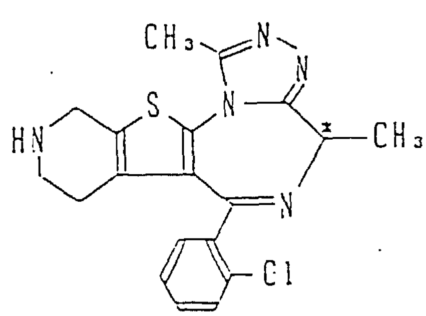

In the same manner as in Preparatory Example 7, dibenzoyl-L-tartaric acid was used to obtain the intended compound.

- $[\alpha]_D^{26}$ +17.56° (C=0.02, EtOH)

Example 5

(+)-6-(2-Chlorophenyl)-3-(1-cyano-1-methylethoxycarbonyl)-8,11-dimethyl-2,3,4,5-tetrahydro-8H-pyrido[4',3':4,5]thieno[3,2-f][1,2,4]triazolo[4,3-a][1,4]diazepine

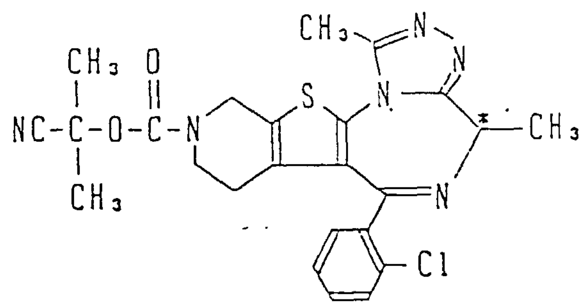

5 g of (-)-6-(2-chlorophenyl)-8,11-dimethyl-2,3,4,5-tetrahydro-8H-pyrido[4',3':4,5]thieno[3,2-f][1,2,4]triazolo[ 4,3-a][1,4]diazepine was dissolved in dichloromethane, to which 5 g of 1-cyano-1-methylethylphenyl carbonate was added, followed by reaction at 100°C for 4 hours while removing the solvent by distillation. After completion of the reaction, the resultant residue was purified by column chromatography (elution solvent: chloroform:methanol = 99:1), thereby obtaining 2.7 g of the intended compound.

- $^1$H-NMR(90 MHz, $CDCl_3$) $\delta$ :
  1.76(s,6H), 1.80 - 2.20(m,2H), 2.10(d,3H), 2.66(s,3H), 3.0 - 3.9(m,2H), 4.24(q,IH), 4.3 - 4.9(m,2H), 7,35(m, 4H)
- FABMS [M+H$^+$] 481
- $[\alpha]_D^{26}$ +17.56° (C=0.02, EtOH)

Example 6

(+)-3-(3-Butynyloxycarbonyl)-6-(2-chlorophenyl)-8,11-dimethyl-2,3,4,5-tetrahydro-8H-pyrido [4',3':4,5]thieno[3,2-f][1,2,4]triazolo[4,3-a][1,4] diazepine

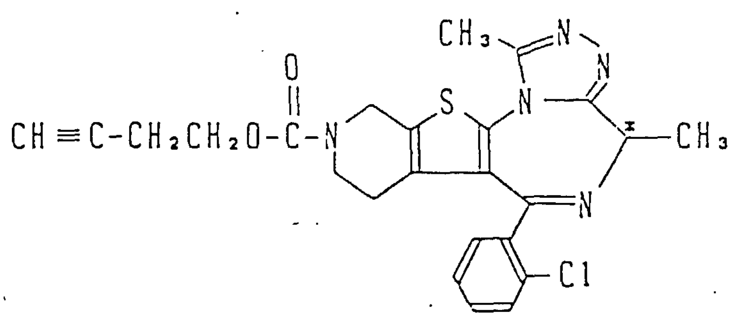

5 g of (-)-6-(2-chlorophenyl)-8,11-dimethyl-2,3,4,5-tetrahydro-8H-pyrido[4';3':4,5]thieno[3,2-f][1,2,4]triazolo[4,3-a][1,4]diazepine was dissolved in dichloromethane, to which 5 g of 3-butynylphenyl carbonate, followed by reaction at 100°C for 4 hours while distilling off the solvent. After completion of the reaction, the resultant residue was purified by column chromatography (elution solvent: dichloromethane:methanol = 99:1), thereby obtaining 1.6 g of the intended compound.

- $^1$H-NMR(90 MHz, $CDCl_3$) $\delta$ :
  7.4(5H,Ar), 4.9(1H,d,J=18Hz,N-$CH_2$[C-2]), 4.5(1H, d, J=18Hz, N-$CH_2$[C-2]), 4.2(1H,n,$C_8$-H), 4.1(2H,t,

J=8Hz,O-$CH_2$), 2.7(3H,s), 2.5(2H,dt,J=1Hz,7Hz,≡-$CH_2$), 2.1(3H,d,J=7Hz,CH$\underline{CH_3}$), 3.0 - 2.0(5H,n)

- $[\alpha]_D^{24}$ +17.0° (C=1, $CHCl_3$)

Example 7

6-(2-Chlorophenyl)-3-cyclopropanecarbonyl-8,11-dimethyl-2,3,4,5-tetrahydro-8H-pyrido[4',3':4,5] thieno[3,2-f][1,2,4] triazolo[4,3-a][1,4]diazepine

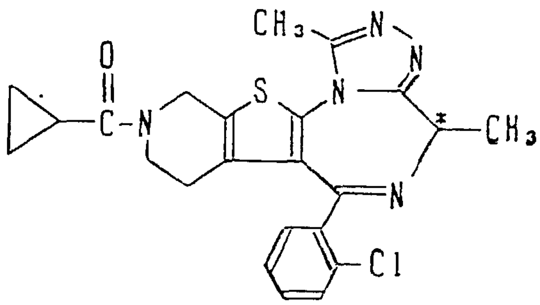

5 g of (-)-6-(2-Chlorophenyl)-3-cyclopropanecarbonyl-8, 11-dimethyl-2,3,4,5-tetrahydro-8H-pyrido[4',3':4,5] thieno[3,2-f][1,2,4]triazolo[4,3-a][1,4]diazepine was dissolved in 70 ml of dichloromethane, to which 1.42 g of triethylamine was added, followed by dropping 1.44 g of cyclopropanecarbonyl chloride under ice-cooling conditions. After completion of the reaction, the reaction solution was washed with a saturated sodium hydrogencarbonate aqueous solution and then with a saturated saline solution, followed by drying with anhydrous magnesium sulfate and removal of the solvent by distillation under reduced pressure. The resultant residue was purified by silica gel column chromatography (elution solvent: dichloromethane:methanol = 98:2), thereby obtaining 4.2 g of the intended compound.

- $^1$H-NMR(90 MHz, $CDCl_3$) δ :
  0.55 - 1.15(m,4H), 1.45 - 2.5(m,3H), 2.10(d,J=6.8Hz,3H), 2.66(s,3H), 2.8 - 4.8(m,3H), 4.26(q,J=6.8Hz,IH), 4.8 - 5.2(m,1H), 7.05 - 7.65(m,4H)
- MS m/z(pos,Fab): 452$(M+H)^+$
- $[\alpha]_D^{26}$ 4.97° (C=1, EtOH)
- $[\alpha]_D^{26}$ 14.91° (C=1, $CHCl_3$)

Other processes of preparing the compounds obtained in the foregoing examples are described in the following.

Preparatory Example 9

1-(Cyano-1-methylethoxycarbonyl)-4-hydroxypiperidine

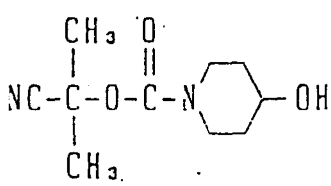

50 g of 1-cyano-1-methylethylphenyl carbonate and 25 g of 4-hydroxypiperidine were heated at 130°C. After completion of the reaction, the resulting product was purified by silica gel column chromatography (elution solvent: hexane:ethyl acetate = 1:1 - 1:2 - 0:1), thereby obtaining 50.5 g of the intended compound.

- $^1H$-NMR(90 MHz, $CDCl_3$) δ :
  1.26 - 2.10(m,5H), 1.80(s,6H), 2.96 - 3.35(m,2H), 3.60 - 4.15(m,3H)

Preparatory Example 10

1-(Cyano-1-methylethoxycarbonyl)-4-piperidone

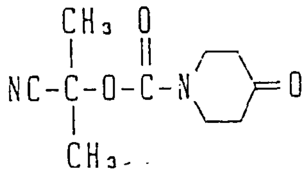

5.06 ml of dimethylsulfoxide was gradually dropped, at -78°C into a solution of 4.15 ml of oxalyl chloride in dichloromethane (50 ml), into which a dichloromethane solution of 5.05 g of 1-(1-cyano-1-methylethoxycarbonyl)-4-hydroxypiperidine was dropped. After agitation at the temperature for 1 hour, 16.57 ml of triethylamine was added, followed by agitation at room temperature for 1 hour. The reaction solution was filtered, washed with water, and dried with anhydrous magnesium sulfate. The solvent was distilled off and the resultant residue was purified by silica gel column chromatography (elution solvent: ethyl acetate:n-hexane = 1:9), thereby obtaining 3.9 g of the intended compound.

- $^1H$-NMR(90 MHz, $CDCl_3$) δ :
  1.80(s,6H), 2.48(t,J=7Hz,4H), 3.74(t,J=7Hz,4H)

Preparatory Example 11

2-Amino-3-(2-chlorobenzoyl)-6-(1-cyano-1-methylethoxycarbonyl)-4,5,6,7-tetrahydro-thieno[2,3-C]pyridine

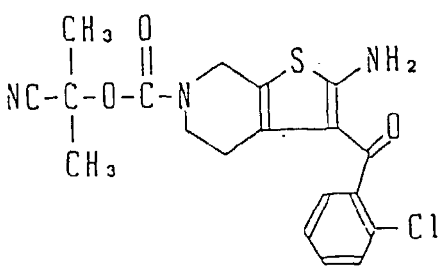

1.6 ml of triethylamine was added to a mixture of 3.9 g of the compound obtained in Preparatory Example 10, 0.6 g of sulfur, 3.3 g of 2-chlorocyanoacetophenone and 20 ml of N,N-dimethylformamide at 40°C and agitated at 60°C for 3 hours. After completion of the reaction, the solvent was evaporated to dryness and washed with ethyl acetate to obtain 5.0 g of the intended compound.

- $^1H$-NMR(90 MHz, $CDCl_3$) δ :
  1.60 - 1.95(m,2H), 1.75(s,6H), 3.40(m,2H), 4.32(m,2H), 7.10 - 7.50(m,6H)

Preparatory Example 12

2-(2-Bromopropionylamino)-3-(2-chlorobenzoyl)-6-(1-cyano-1-methylethoxycarbonyl)-4,5,6,7-tetrahydrothieno [2,3-C]pyridine

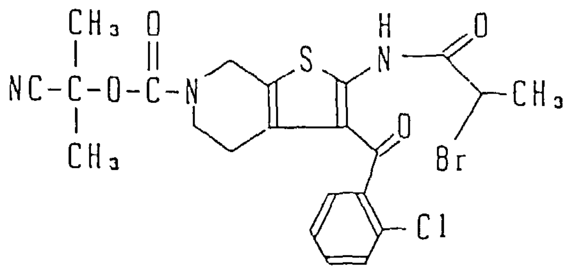

4.6 g of 2-bromopropionyl bromide was dropped into a mixture of 5.0 g of the compound obtained in Preparatory Example 11, 2.1 g of sodium hydrogencarbonate, 50 ml of water and 200 ml of toluene at 60°C. After completion of the reaction, ethyl acetate was added, from which the aqueous phase was removed. The organic phase was washed with a saturated saline solution and dried with anhydrous magnesium sulfate. The solvent was distilled off to obtain 6.0 g of the intended compound.

- $^1$H-NMR(90 MHz, $CDCl_3$) δ :
  1.76(s,6H), 1.88(m,2H), 2.00(d, J=7Hz, 3H), 3.24 - 3.60(m,2H), 4.20 - 4.68(m,2H), 4.62(q, J=7Hz, 1H), 7.00 - 7.50(m,4H)

Preparatory Example 13

2-(2-Aminopropionylamino)-3-(2-chlorobenzoyl)-6-(1-cyano-1-methylethoxycarbonyl)-5,6,7,8-tetrahydrothieno[2,3-C] pyridine

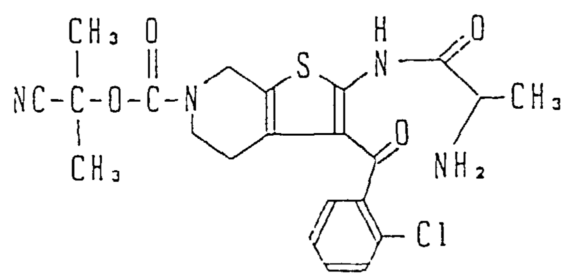

6.0 g of the compound obtained in Preparatory Example 12 was dissolved in 50 ml of ethyl acetate, into which ammonia was introduced at -20°C for 2 hours, followed by agitation in a sealed tube at 100°C for 5 hours. After completion of the reaction, the reaction product was extracted with 2N hydrochloric acid and the resultant aqueous phase was neutralized with sodium hydrogencarbonate, which was subsequently saturated with sodium chloride and extracted with chloroform. After drying with anhydrous magnesium sulfate, the solvent was distilled off to obtain 0.7 g of the intended compound.

- $^1$H-NMR(90 MHz, $CDCl_3$) δ :
  1.51(d, J=7Hz,3H), 1.50 - 2.04(m,2H), 1.78(s,6H), 3.28 - 3.60(m,2H), 3.62 - 3.96(m,1H), 4.50(m,2H), 7.20 - 7.54(m,4H)

Preparatory Example 14

3-Methyl-5-(2-chlorophenyl)-8-(1-cyano-1-methylethoxycarbonyl)-6,7,8,9-tetrahydro-1H,3H-pyrido [4',3':4,5]thieno [2,3-e][1,4]diazepin-2-one

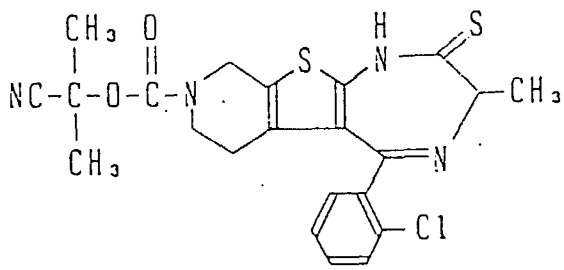

A mixture of 0.4 g of the compound obtained in Preparatory Example 13, 0.7 g of phosphorus pentasulfide, 0.4 g of sodium hydrogencarbonate and 40 ml of 1,2-dimethoxyethane was refluxed for 2 hours. After completion of the reaction, the solvent was distilled off, to which methanol was added, followed by removal of insoluble matters by filtration and concentration. The residue was purified by silica gel column chromatography (elution solvent: chloroform: methanol = 99:1), thereby obtaining 0.3 g of the intended compound.

- $^1$H-NMR(90 MHz, $CDCl_3$) δ :
  1.50 - 2.0(m,2H), 1.76(s,6H), 1.92(d,J=7Hz,3H), 3.0 - 4.0(m,2H), 4.0 - 4.3(m,IH), 4.3 - 5.0(m,2H), 7.1 - 7.6 (m,4H)

Example 8

3-(1-Cyano-1-methylethoxycarbonyl)-6-(2-chlorophenyl)-8,11-dimethyl-2,3,4,5-tetrahydro-8H-pyrido[4',3':4,5] thieno [3,2-f][1,2,4]triazolo[4,3-a][1,4]diazepine

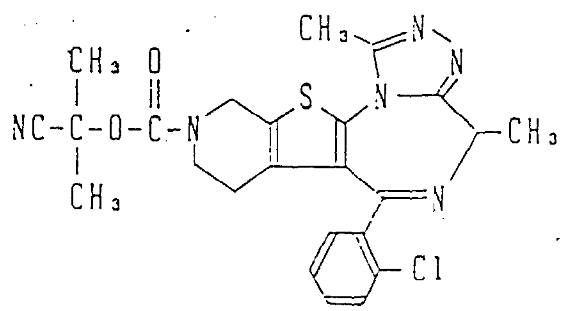

0.3 g of the compound obtained in Preparatory Example 14 and 0.3 g of acetohydrazide were dissolved in 20 ml of 1,4-dioxane and refluxed for 3 hours. After completion of the reaction, the solvent was distilled off and the residue was purified by silica gel column chromatography (elution solvent: chloroform:methanol = 99:1), thereby obtaining 0.20 g of the intended compound.

- $^1$H-NMR(90 MHz, $CDCl_3$) δ :
  1.76(s,6H), 1.80 - 2.20(m,2H), 2.10(d,3H), 2.66(s,3H), 3.0 - 3.9(m,2H), 4.24(q,IH), 4.3 - 4.9(m,2H), 7.35(m, 4H)

Preparatory Example 15

N-(3-Butynyloxycarbonyl)-4-hydroxypiperidine

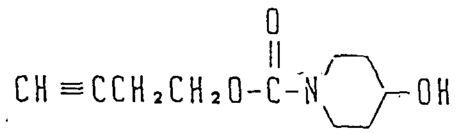

10.0 g of 3-butynylphenyl carbonate and 5.8 g of 4-hydroxypiperidine were heated in a solvent-free condition at 100°C of 30 minutes. After completion of the reaction, silica gel column chromatography (elution solvent: hexane: ethyl acetate = 1:1 - 1:2) was used for purification to obtain 10.6 g of the intended compound.

- $^1$H-NMR(90 MHz, $CDCl_3$) δ :
  1.16 - 2.1(m,5H), 1.98(t,J=2Hz,1H), 1.42(dt,J=2Hz,7Hz,2H), 2.9 - 3.5(m,2H), 3.6 - 4.1(m,3H), 4.15(t,J=7Hz, 2H)

Preparatory Example 16

N-(3-Butynyloxycarbonyl)-4-piperidone

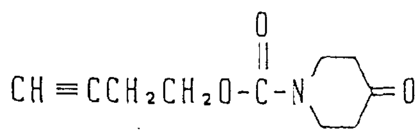

25 ml of oxalyl chloride was added to 500 ml of dichloromethane, into which 41 ml of dimethyl sulfoxide was gradually dropped in a stream of nitrogen at a temperature of from -50°C to -70°C. Subsequently, 10.3 g of N-(3-butynyloxycarbonyl)-4-hydroxypiperidine was dissolved in 50 ml of dichloromethane, followed by gradually dropping into the reaction mixture. Finally, 120 ml of triethylamine was dropped, followed by gradually increasing the temperature up to room temperature. The reaction solution was charged into a saturated saline solution, extracted three times with dichloromethane and dried with anhydrous magnesium sulfate, after which the solvent was distilled off under reduced pressure. The resultant residue was purified by the use of silica gel column chromatography (elution solvent: hexane: ethyl acetate = 3:1), thereby obtaining 8.9 g of the intended compound.

- $^1$H-NMR(90 MHz, $CDCl_3$) δ :
  2.0(t,J=2Hz,lH), 2.3 - 2.8(m,6H), 3.76(t,J=7Hz,4H), 4.21(t,J=7Hz, 2H)

Preparatory Example 17

2-Amino-3-(2-chlorobenzoyl)-6-(3-butynyloxycarbonyl)-4,5,6,7-tetrahydro-thieno[2,3-C]pyridine

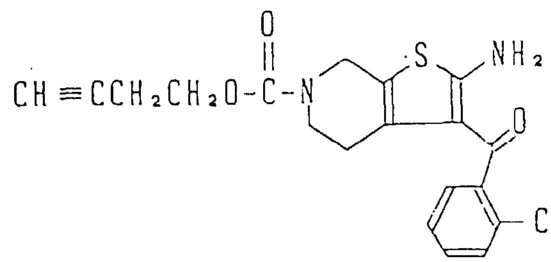

7.4 g of N-(3-butynyloxycarbonyl)-4-piperidone, 1.21 g of sulfur and 61.5 g of 2-chlorocyanoacetophenone were dissolved in 25 ml of dimethylformamide, to which 3.5 ml of triethylamine was further added, followed by agitation at 60°C for 1 hour. After completion of the reaction, silica gel column chromatography )elution solvent: dichloromethane: methanol = 99:1) was used for purification, thereby obtaining 11.2 g of the intended compound.

- $^{1}$H-NMR(90 MHz, $CDCl_3$) δ :
  1.64 - 1.90(m,2H), 1.96(t,J=2Hz,IH), 2.3 - 2.7(t,J=2Hz, 7Hz, 2H), 3.4(t,J=7Hz,2H), 4.14(t,J=7Hz, 2H), 4.3 - 4.5(m,2H), 7.0 - 7.5(m,6H)

Preparatory Example 18

2-(2-Bromopropionylamino)-3-(2-chlorobenzoyl)-6-(3-butynyloxycarbonyl)-4,5,6,7-tetrahydrothieno[2,3-C]pyridine

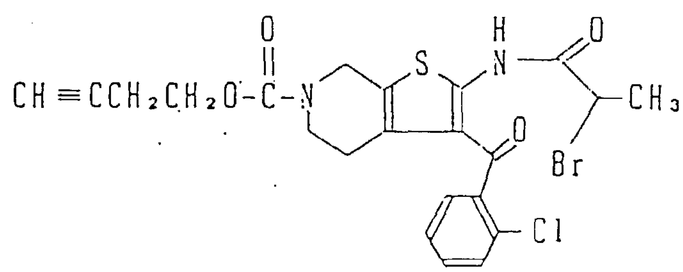

1.35 g of 2-amino-3-(2-chlorobenzoyl)-6-(3-butynyloxycarbonyl)-4,5,6,7-tetrahydro-thieno[2,3-C]pyridine was dissolved in 20 ml of dioxane, to which 0.33 g of pyridine was added, followed by dropping at 0°C 0.90 g of 2-bromopropionyl bromide. After completion of the reaction, the reaction mixture was charged into water, extracted with dichloromethane and dried with anhydrous magnesium sulfate, after which the solvent was distilled off under reduced pressure. The resultant residue was purified by silica gel column chromatography (elution solvent: dichloromethane:hexane = 1:1 - 1:0), thereby obtaining 1.19 g of the intended compound.

- $^{1}$H-NMR(90 MHz, $CDCl_3$) δ :
  2.02(t,J=7Hz,3H), 1.7 - 2.2(m,3H), 3.5(dt,J=2Hz, 7Hz, 2H), 3.44(t,J=7Hz,2H), 4.16(t,J=7Hz, 2H), 4.4 - 4.8 (m,3H), 7.0 - 7.5(m,5H)

Preparatory Example 19

2-(2-Aminopropionylamino)-3-(2-chlorobenzoyl)-6-(3-butynyloxycarbonyl)-4,5,6,7-tetrahydrothieno[2,3-C]pyridine

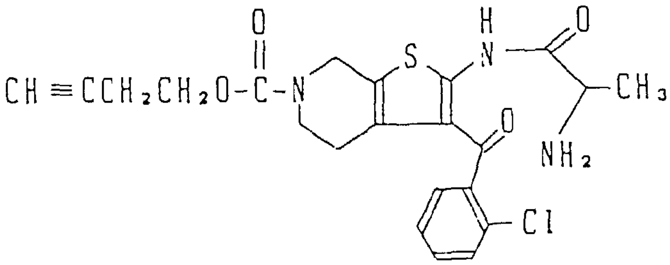

1.16 g of 2-(2-bromopropionylamino)-3-(2-chlorobenzoyl)-6-(3-butynyloxycarbonyl)-4,5,6,7-tetrahydrothieno[2,3-C]pyridine was dissolved in 36 ml of ethyl acetate, into which ammonia gas was introduced under cooling conditions, followed by heating in a sealed tube at 100°C. After completion of the reaction, the mixture was cooled, to which 50 ml of ethyl acetate was added. The mixture was washed with 1N hydrochloric acid, after which the aqueous phase was neutralized with a sodium carbonate aqueous solution and extracted with chloroform. The resultant organic phase was dried with anhydrous magnesium sulfate. The solvent was distilled off under reduced conditions and the resultant residue was purified by the use of silica gel column chromatography (elution solvent: dichloromethane), thereby obtaining 0.36 g of the intended compound.

- $^1$H-NMR(90 MHz, $CDCl_3$) δ :
  1.5(t,J=7Hz,3H), 1.6 - 1.8(brs,2H), 1.8 - 2.1(m,3H), 2.52(dt,J=2Hz,7Hz,2H), 3.44(t,J=7Hz,2H), 3.76(q,J=7Hz, 1H), 4.16(t,J=7Hz, 2H), 4.5 - 4.64(m,2H), 7.1 - 7.7(m,5H)

Preparatory Example 20

3-Methyl-5-(2-chlorophenyl)-8-(3-butynyloxycarbonyl)-6,7,8,9-tetrahydro-1H,3H-pyrido[4',3':4,5] thieno[3,2-f][1,4] diazepin-2-one

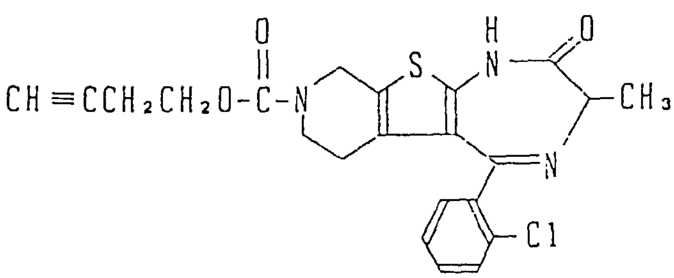

0.36 g of 2-(2-aminopropionylamino)-3-(2-chlorobenzoyl)-6-(3-butynyloxycarbonyl)-4,5,6,7-tetrahydro[2, 3-C]pyridine was dissolved in 10 ml of toluene and 0.8 ml of pyridine, to which 0.18 ml of acetic acid, followed by refluxing while removing the resultant water. After completion of the reaction, the toluene was distilled off under reduced pressure and dichloromethane was added to the distilled reaction solution, followed by washing with water and drying with anhydrous magnesium sulfate. The solvent was distilled off under reduced pressure and the resultant residue was purified by the use of silica gel column chromatography (elution solvent)dichloromethane:methanol = 100:0 - 97:3), thereby obtaining 0.22 g of the intended compound.

- $^1$H-NMR(90 MHz, $CDCl_3$) δ :

1.76(d,J=7Hz,3H), 1.6 - 2.2(m,3H), 2.5(dt,J=2Hz,7Hz,2H), 2.9 - 4.0(m,2H), 3.86(q,J=7Hz,1H), 4.17(t,J=7Hz, 2H), 4.3 - 4.9(m,2H), 7.0 - 7.6(m,5H)

Preparatory Example 21

3-Methyl-5-(2-chlorophenyl)-8-(3-butynyloxycarbonyl)-6,7,8,9-tetrahydro-1H,3H-pyrido[4',3':4,5] thieno[3,2-f][1,4] diazepin-2-thione

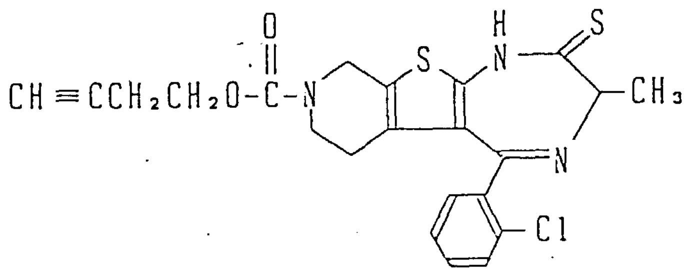

0.21 g of 3-methyl-5-(2-chlorophenyl)-8-(3-butynyloxycarbonyl)-6,7,8,9-tetrahydro-1H,3H-pyrido[4',3':4,5] thieno[3,2-f][1,4]diazepin-2-one was dissolved in 10 ml of dimethoxyethane, to which 0.11 g of sodium hydrogencarbonate and 0.22 g of phosphorus pentasulfide were added, followed by heating at 80°C for 3 hours. After completion of the reaction, dichloromethane and methanol were added, and the mixture was filtered, followed by addition of silica gel to the resultant filtrate and evaporating the solvent to dryness. Silica gel column chromatography (elution solvent: dichloromethane:methanol = 99:1) was used for purification, thereby obtaining 0.15 g of the intended compound.

- $^1$H-NMR(90 MHz, $CDCl_3$) $\delta$ :

  1.12 - 2.00(m,2H), 1.73(d,J=7Hz,3H), 2.12(t,J=2Hz,1H), 2.40(dt,J=2Hz,7Hz,2H), 2.64 - 3.80(m,2H), 4.01(q, J=7Hz,IH), 4.02(t,J=7Hz, 2H), 4.10 - 4.76(m,2H), 7.28(m,2H)

Example 9

3-(3-Butynyloxycarbonyl)-6-(2-chlorophenyl)-8,11-dimethyl-2,3,4,5-tetrahydro-8H-pyrido[4',3':4,5] thieno[3,2-f][1,2,4] triazolo[4,3-a][1,4]diazepine

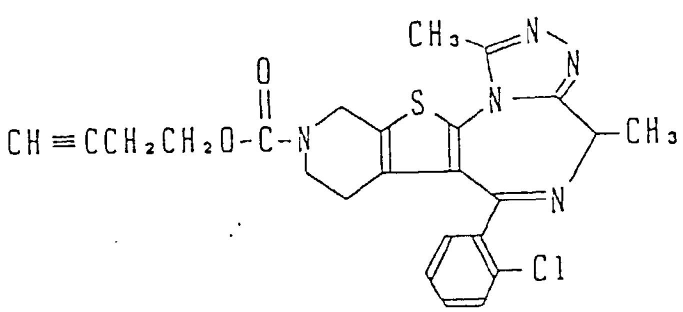

100 mg of acetohydrazide was added to 150 mg of 3-methyl-5-(2-chlorophenyl)-8-(3-butynyloxycarbonyl)-6,7,8,9-tetrahydro-1H,3H-pyrido[4',3':4,5]thieno[3,2-f][1,4] diazepin-2-thione, to which 2 ml of dioxane was further added, followed by heating at 130°C for 3 hours while distilling off the solvent. After completion of the reaction, the resultant residue was purified by the use of silica gel column chromatography (elution solvent: dichloromethane:methanol = 98: 2), thereby obtaining 80 mg of the intended compound.

- $^1H$-NMR(90 MHz, $CDCl_3$) δ :
  7.5(4H,Ar), 4.9(1H,d,J=18Hz,N-$CH_2$[C-2:]), 4.5(1H,d,J=18Hz,N-$CH_2$[C-2:]), 4.2(1H,m,$C_8$-H), 4.1(2H,t, J=8Hz,0-$CH_2$), 2.7(3H,s), 2.5(2H,dt,J=1Hz,7Hz, ≡-$CH_2$), 2.1(3H, d,J=7Hz,CHCH), 3.0 - 2.0(5H,m)

Preparatory Example 22

1-Cyclopropanecarbonyl-4-hydroxypiperidine

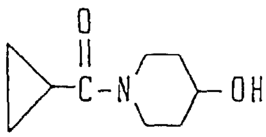

20 g of 4-hydroxypiperidine was dissolved in 400 ml of dichloromethane, to which 24 g of triethylamine was added. At -60°C, 100 ml of a dichloromethane solution containing 20.7 g of cyclopropanecarbonyl chloride was further added. After completion of the reaction, the reaction solution was extracted with chloroform under salting-out conditions and dried with anhydrous magnesium sulfate, and the solvent was distilled off under reduced pressure. The resultant residue was purified by the use of silica gel column chromatography (elution solvent: dichloroethane), thereby obtaining 32 g of the intended compound (yield 96%).

- $^1H$-NMR(90 MHz, $CDCl_3$) δ :
  0.55 - 1.55(m,4H), 1.15 - 2.15(m,5H), 2.4(bs,1H), 2.8 - 3.55(m,2H), 3.65 - 4.3(m,3H)

Preparatory Example 23

1-Cyclopropanecarbonyl-4-piperidone

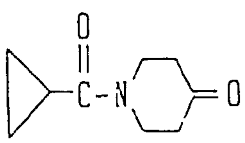

66 g of oxalic chloride was dissolved in 500 ml of dichloroethane, into which 61 g of dimethyl sulfoxide was gradually dropped at -67°C. 44 g of l-cycloprpanecarbonyl-4-hydroxypiperidine dissolved in 200 ml of dichloromethane was dropped into the above solution at -67°C. At -67°C, 131 g of triethylamine was further added, followed by returning to room temperature. The resultant salt was removed by filtration and the filtrate was once concentrated, after which water was added to the concentrate, followed by extraction with ethyl acetate and drying with anhydrous magnesium sulfate. Moreover, the aqueous phase was extracted with chloroform and dried similarly. The solvent was distilled off under reduced pressure and the resultant residue was purified by the use of silica gel column chromatography (elution solvent: ethyl acetate:hexane = 3:7), thereby obtaining 33 g of the intended compound (yield 76%).

- $^1H$-NMR(90 MHz, $CDCl_3$) δ :
  0.65 - 1.2(m,4H), 1.6 - 2.0(m,1H), 2.49(t,J=6.1Hz,4H), 3.91(t, J=6.1Hz,4H)

Preparatory Example 24

2-Amino-3-(2-chlorobenzoyl)-6-cyclopropanecarbonyl-4,5,6,7-tetrahydro-thieno[2,3-C]pyridine

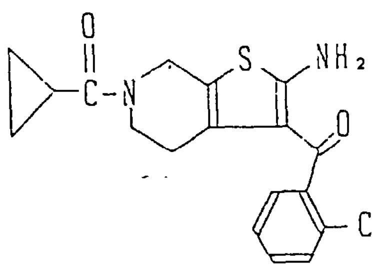

33 g of 1-cyclopropanecarbonyl-4-piperidone, 6.3 g of sulfur and 25.5 g of 2-chloro-cyanoacetophenone were dissolved in 330 ml of N,N-dimethylformamide, to which 20 g of triethylamine was added at 60°C. After completion of the reaction, the solvent was distilled off under reduced pressure and methanol was added to the resultant residue for crystallization. The crystals were filtered and washed with methanol to obtain 49.4 g of the intended compound (yield 69%).

- $^1$H-NMR(90 MHz, $CDCl_3$) δ :
  0.55 - 1.15(m,4H), 1.4 - 2.0(m,3H), 3.35 - 3.75(m,2H), 4.3 - 4.7(m,2H), 7.0 - 7.7(m,4H)

Preparatory Example 25

2-(2-Bromopropionylamino)-3-(2-chlorobenzoyl)-6-cyclopropanecarbonyl-4,5,6,7-tetrahydrothieno[2,3-C]pyridine

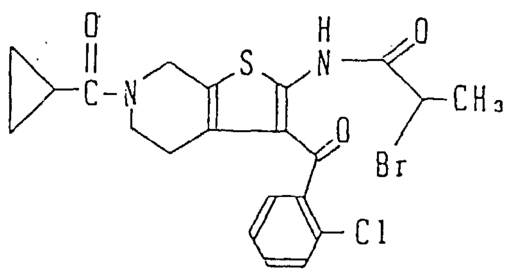

450 ml of toluene and 150 ml of water were added to 21.83 g of 2-amino-3-(2-chlorobenzoyl)-6-cyclopropanecarbonyl-4,5,6,7-tetrahydro-thieno [2,3-C]pyridine. 10.16 g of sodium hydrogencarbonate was further added, to which 9.5 ml of 2-bromopropionyl bromide was added while heating to 50 to 60°C. Moreover, a sodium hydrogencarbonate aqueous solution (10.6 g of sodium hydrogencarbonate and 150 ml of waterl) and 5 ml of 2-bromopropionyl bromide were added to complete the reaction. After the completion of the reaction, ethyl acetate was added and the reaction solution was washed once with a saturated saline solution and dried with anhydrous magnesium sulfate, and the solvent was distilled off under reduced pressure to obtain 29.9 g of the intended compound (at a quantitative yield).

- $^1$H-NMR(90 MHz, $CDCl_3$) δ :
  0.55 - 1.2(m,4H), 1.6 - 2.2(m,3H), 1.99(d,J=7.2Hz,3H), 3.35 - 3.8(m,2H), 4.45 - 4.85(m,2H), 4.61(q,J=7.2Hz, IH), 7.0 - 7.6(m,4H)

Preparatory Example 26

2-(2-Aminopropionylamino)-3-(2-chlorobenzoyl)-6-cyclopropanecarbonyl-4,5,6,7-tetrahydro-thieno-[2,3-C]pyridine

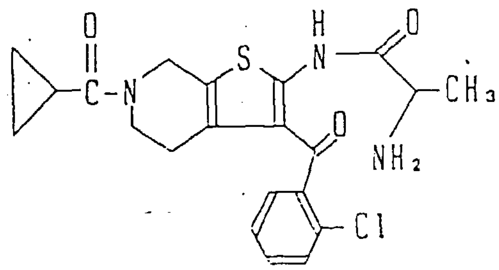

23.04 g of 2-(2-bromopropionylamino)-3-(2-chlorobenzoyl)-6-cyclopropanecarbonyl-4,5,6,7-tetrahydrothieno [2,3-C]pyridine was dissolved in 65 ml of 1,2dichloroethane and 65'.ml of ethyl acetate,into which ammonia gas was passed At -15°C for 1 hour. This solution was placed in a sealed tube and reacted at 110°C for 2 hours. In order to complete the reaction, ammonia was again passed into the solution at -15°C for 30 minutes in the sealed tube where the reaction was continued at 110°C for 1.5 hours. After ice-cooling, the reaction solution was charged into ice-cooled 2N hydrochloric acid, to which ethyl acetate was added, from which the resultant aqueous phase was collected. Sodium carbonate was added to the aqueous phase under ice-cooling conditions so that the pH was adjusted to 8, followed by extraction with chloroform under salting-out conditions. The extract was washed with a saturated saline solution and dried with anhydrous magnesium sulfate, followed by filtration and concentration to obtain 12.97 g (yield 64%) of the intended compound.

- $^1$H-NMR(90 MHz, $CDCl_3$) δ :
  0.45 - 1.2(m,4H), 1.48(d,J=7.2Hz,3H), 1.4 - 2.4(m,3H), 3.35 - 3.85(m,2H), 3.74(q,J=7.2Hz,IH), 4.45 - 4.85 (m,2H), 7.0 - 7.7(m,4H)

Preparatory Example 27

5-(2-Chlorophenyl)-8-cyclopropanecarbonyl-3-methyl-6,7,8,9-tetrahydro-1H,3H-pyrido[4',3':4,5] thieno[3,2-f][1,4] diazepin-2-one

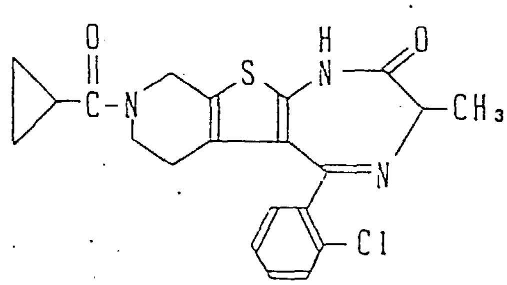

12.95 g of 2-(2-aminopropionylamino)-3-(2-chlorobenzoyl)-6-cyclopropanecarbonyl-4,5,6,7-tetrahydro-thieno[2,3-C]pyridine was dissolved in 260 ml of toluene and 90 ml of pyridine, to which 5.4 g of acetic acid was added, followed by heating under reflux for 5 hours. After removal of the solvent by distillation, benzene was added and the resultant crystals were collected by filtration to obtain 2.96 g of the intended compound. The mother liquor was subjected to silica gel column chromatography (elution solvent: ethyl acetate:hexane = 4:6) to obtain 3.84 g of the intended compound.

- $^1$H-NMR(90 MHz, $CDCl_3$) δ :

0.5 - 1.25(m,4H), 1.3 - 2.3(m,3H), 1.75(d,J=6.5Hz,3H), 2.8 - 5.25(m,5H), 7.0 - 7.65(m,4H)

Preparatory Example 28

5-(2-Chlorophenyl)-8-cyclopropanethiocarbonyl-3-methyl-6,7,8,9-tetrahydro-1H,3H-pyrido[4',3':4,5] thieno[3,2-f][1,4] diazepin-2-thione

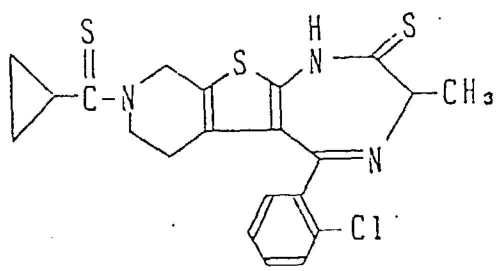

2.92 g of 5-(2-chlorophenyl)-8-cyclopropanecarbonyl-3-methyl-6,7,8,9-tetrahydro-1H,3H-pyrido[4',3':4,5] thieno[3,2-f][1,4]diazepin-2-one was suspended in 60 ml of 1,2-dimethoxyethane, to which 1.78 g of sodium hydrogencarbonate and 3.92 g of phosphorus pentasulfide were added, followed by heating under reflux for 4 hours. The reaction solution was filtered through Celite and and filter cake was washed sufficiently with 30% methanol-dichloromethane and combined with the filtrate. The combined filtrate was concentrated and subjected to silica gel column chromatography (elution solvent:
dichloromethane) to obtain 1.03 g of the intended compound (yield 33%).

- $^1$H-NMR(90 MHz, 10%$CD_3OD$-$CDCl_3$) δ :
  0.8 - 1.55(m,4H), 1.6 2.75(m,3H), 2.00(d,J=6.1Hz,3H), 3.2 - 5.2 and 5.6 - 6.2(each m, total 5H), 7.2 - 7.8(m, 4H)
- MS m/z(Pos. FAB): 446(M+H)$^+$

Preparatory Example 29

6-(2-Chlorophenyl)-3-cyclopropanethiocarbonyl-8,11-dimethyl-2,3,4,5-tetrahydro-8H-pyrido[4',3':4,5] thieno[3,2-f] [1,2,4]triazolo[4,3-a][1,4]diazepine

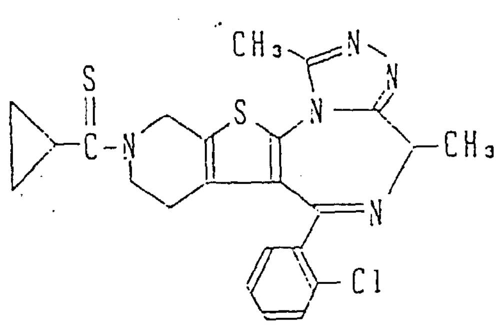

1.00 g of 5-(2-Chlorophenyl)-8-cyclopropanethiocarbonyl-3-methyl-6,7,8,9-tetrahydro-1H,3H-pyrido[4',3':4,5] thieno[3,2-f][1,4]diazepin-2-thione was dissolved in 40 ml of dioxane, to which 0.17 g of acetohydrazide was added, followed by agitation at an ambient temperature of 90°C for 10 hours and then at 120°C for 1 hour. 0.17 g of acetohydrazide was further added, followed by further agitation at 120°C for 1 hour to complete the reaction. After removal of the solvent by distillation, the residue was subjected to silica gel column chromatography (elution solvent:dichloromethane:methanol = 99:1) to obtain 280 mg of the intended compound (yield 27%).

- $^1$H-NMR(90MHz, $CDCl_3$) δ :
  0.75 - 1.75(m,4H), 1.75 - 2.6(m,3H), 2.10(d,J=6.8Hz,3H), 2.67(s,3H), 3.2 - 4.6(m,2H), 4.26(q,J=6.8Hz,IH), 4.65 - 5.4 and 5.55 - 6.0(each m, total 2H), 7.0 - 7.65(m,4H)

Example 10

6-(2-Chlorophenyl)-3-cyclopropanecarbonyl-8,11-dimethyl-2,3,4,5-tetrahydro-8H-pyrido[4',3':4,5]thieno[3,2-f] [1,2,4] triazolo[4,3-a][1,4]diazepine

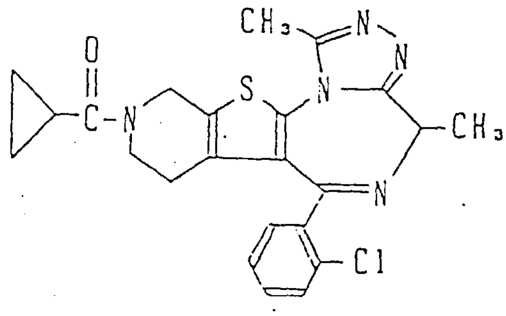

100 mg of 6-(2-chlorophenyl)-3-cyclopropanethiocarbonyl-8,11-dimethyl-2,3,4,5-tetrahydro-8H-pyrido[4',3':4,5] thieno[3,2-f][1,2,4]triazolo[4,3-a][1,4]diazepine was dissolved in 10 ml of dichloromethane, to which 10 ml of 4N hydrochloric acid was added, followed by further addition of 1 ml of an aqueous solution containing 30 mg of sodium nitrite under agitation. The solution was ice-cooled, to which sodium carbonate was added for adjustment of the pH to 8, followed by extraction with dichloromethane, washing with a saturated saline solution and drying with anhydrous magnesium sulfate. The solution was filtered and a concentrated residue was subjected to silica gel column chromatography (elution solvent: dichloromethane:methanol = 99:1), thereby obtaining 69.9 mg of the intended compound (yield 72%).

- $^1$H-NMR(90MHz, $CDCl_3$) δ :
  0.55 - 1.15(m,4H), 1.45 - 2.5(m,3H), 2.10(d,J=6.8Hz,3H), 2.66(s,3H), 2.8 - 4.8(m,3H), 4.26(q,J=6.8Hz,IH), 4.8 - 5.2 (m,IH), 7.05 - 7.65(m,4H)
- MS m/z(Pos. FAB): 452$(M+H)^+$

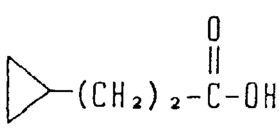

100 ml of methanol, 100 ml of tetrahydrofuran and 2 g of 10% palladium-carbon (containing 50% water) were added to 5.06 g of ethyl 3-cyclopropylacrylate, followed by hydrogenation reaction overnight at normal temperature and normal pressure conditions. The catalyst was removed by filtration and the solvent was distilled off. 20 ml of methanol, 20 ml of tetrahydrofuran, 10 ml of water and 7 g of sodium hydroxide were added to the residue and agitated at 80°C for 3.5 hours. The solvent was distilled off, to which water was added, followed by washing with ethyl acetate. A hydrochloric acid aqueous solution was added to the resultant aqueous phase under ice-cooling conditions to adjust the pH to 3, followed by extraction with chloroform under slating-out conditions and drying with anhydrous magnesium sulfate. This was filtered and, after removal of the solvent by distillation, the resulting residue was subjected to silica gel column chromatography (developing solvent: dichloromethane) to obtain 1.80 g of the intended compound.

- $^1$H-NMR(90MHz, $CDCl_3$) δ :
  0.65 - 1.1(m,2H), 1.1 - 1.85(m,5H), 2.33(t, J=7.2Hz,2H), 8.9(bs,1H)

Preparatory Example 30

Ethyl tetrahydropyrane- Δ 4, a-acetate

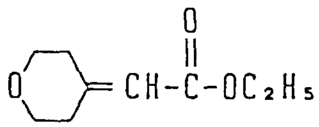

1.2 g (30 mmols) of sodium hydride was added to 100 ml of a dimethylformamide solution of 6.72 g (30 mmols) of diethylphosphonoethyl acetate under ice-cooling conditions, and was agitated for 10 minutes. 2.5 g (25 mmols) of tetrahydro-4H-pyran-4-one was further added to the mixture under ice-cooling conditions and was returned to room temperature, followed by agitation at 80°C for 2 hours. After completion of the reaction, ethyl acetate was added, followed by washing with a saturated saline solution and drying with magnesium sulfate. This was concentrated under reduced pressure and the resultant residue was subjected to silica gel column chromatography (elution solvent: hexane: ethyl acetate = 9:1) thereby obtaining 4.2 g of the intended compound as a light yellow oily substance.

- $^1H$-NMR($CDCl_3$) δ :
  1.3(t,J=7,2Hz,3H), 2.0 - 2.3(m,2H), 3.0(bs,2H), 3.8(t,J=5.4Hz,2H), 4.0 - 4.3(m,2H), 4.1(q,J=7.2Hz,2H), 5.6 (bs,1H)

Preparatory Example 31

Ethyl 4-tetrahydropyranylacetate

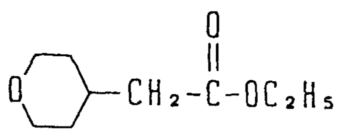

2.0 g of ethyl tetrahydropyran- Δ 4, a-acetate was dissolved in 50 ml of methanol, to which 10% palladium-carbon was added, followed by hydrogenation for 3 hours. After removal of the catalyst by filtration, the reaction mixture was concentrated under reduced pressure to obtain 1.6 g of the intended compound.

- $^1H$-NMR($CDCl_3$) δ :
  1.1 - 2.3(m,7H), 1.3(t,J=7,2Hz,3H), 3.2 - 3.6(td,J=12,6Hz,2.9Hz,2H), 3.8 - 4.3(m,2H), 4.1(q,J=7.2Hz,2H)

Preparatory Example 32

4-Tetrahydropyranylacetic acid

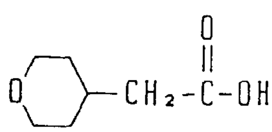

20 ml of methanol, 10 ml of water and 1 g of sodium hydroxide were added to 0.9 g of ethyl 4-tetrahydropyranylacetate and agitated at 80°C for 1 hour. The solvent was removed by distillation, to which water was added. After washing with ethyl acetate, a hydrochloric acid aqueous solution was added to the resultant aqueous phase to an

extent of pH of 3, followed by extraction with chloroform under salting-out conditions and drying with anhydrous magnesium sulfate. This was removed by filtration and the solvent was distilled off, thereby obtaining 0.87 g of a crude intended compound.

- $^{1}H$-NMR($CDCl_3$) δ :
  1.0 - 2.4(m,5H), 2.28(bd,J=6.5Hz,2H), 3.37(td,J-11.5Hz,2.9Hz,2H), 3.7 - 4.1(m,2H). 7.85(bs,1H)

Preparatory Example 33

Tetrahydropyran- $\Delta^{4,a}$-acetic acid

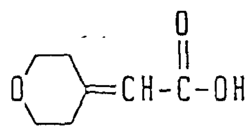

20 ml of methanol, 10 ml of water and 1 g of sodium hydroxide were added to 0.8 g of ethyl tetrahydropyran-$\Delta^{4,a}$-acetate and agitated at 80°C for 2 hours. After removal of the solvent by distillation, water was added to the mixture, followed by washing with ethyl acetate. A hydrochloric acid aqueous solution was added to the aqueous phase to render it acidic, followed by extraction with chloroform under salting-out conditions and drying with anhydrous magnesium sulfate. This was filtered off and the solvent was distilled off, followed by subjecting the resultant residue to silica gel column chromatography (developing solvent: dichloromethane) thereby obtaining 0.17 g of the intended compound (yield 25%).

- $^{1}H$-NMR($CD_3OD$-$CDCl_3$) δ :
  2.34(bt, J=5.4Hz, 2H), 2.98(bt,J=5.4Hz,2H), 3.5 - 3.95(m,4H), 5.66(bs,IH), 8.45(bs,1H)

Example 11

6-(2-Chlorophenyl)-3-[(trans)-3-cyclopropyl]acryloyl-8,11-dimethyl-2,3,4,5-tetrahydro-8H-pyrido[4',3':4,5] thieno[3,2-f] [1,2,4]triazolo[4,3-a][1,4]diazepine

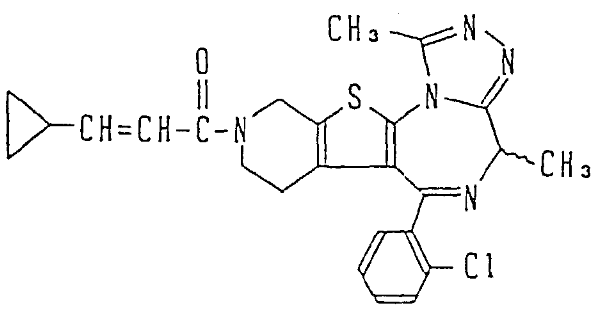

(1) Preparation of ethyl (trans)-3-cyclopropylacrylate

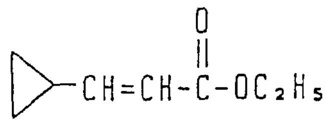

38.38 g of ethyl diethylphosphonoacetate was dissolved in 300 ml of N,N-dimethylformamide, to which 6.85 g of 60% sodium hydride at 0°C, followed by agitation at room temperature for 30 minutes and dropping 10 g of cyclopropanealdehyde at 0°C. After agitation at room temperature for 2 hours, iced water was added, followed by extraction with ether, washing with water and drying with anhydrous magnesium sulfate. This was removed by filtration and a concentrated residue was subjected to silica gel column chromatography (developing solvent: ethyl acetate:hexane = 2:98), thereby obtaining 11.18 g of the intended compound as a trans product (yield 60%).

- $^1$H-NMR($CDCl_3$) δ :
  0.4 - 1.1(m,4H), 1.26(t,J=7.2Hz, 3H), 1.3 - 1.8(m,1H), 4.13(q,J=7.2Hz,2H), 5.82(d,J=15.5Hz,1H), 6.37(dd, J=15.5Hz, 10.1Hz,1H)

(2) Preparation of (trans)-3-cyclopropylacrylic acid

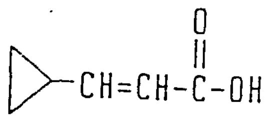

100 ml of methanol, 50 ml of water and 6.4 g of sodium hydroxide were added to 11.18 g of ethyl (trans)-3-cyclopropylacrylate obtained in the above method and subjected to reaction at 80°C for 1.5 hours. After concentration, concentrated hydrochloric acid was added so as to render the reaction mixture acidic, followed by extraction with chloroform, washing with a saturated saline solution and drying with anhydrous magnesium sulfate. This sulfate was removed by filtration and the filtrate was concentrated to obtain 8.82 g of the intended compound (yield 99%).

- $^1$H-NMR($CDCl_3$) δ :
  0.3 - 1.2(m,4H), 1.2 - 1.9(m,1H), 5.83(d,J=15.1Hz,1H), 6.47(dd,J=15.1Hz, 6.5Hz,1H), 9.06(bs,1H)

(3) 6-(2-Chlorophenyl)-3-[(trans)-3-cyclopropyl] acryloyl-8,11-dimethyl-2,3,4,5-tetrahydro-8H-pyrido[4', 3':4,5]thieno [3,2-f-][1,2,4]triazolo[4,3-a] [1,4]diazepine

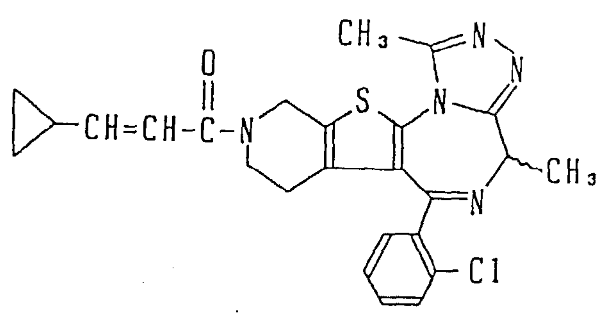

90 mg of (trans)-3-cyclopropylacrylic acid, 120 g of 1-hydroxybenzotriazole · monohydrate and 240 mg of 6-(2-chlorophenyl)-8,11-dimethyl-2,3,4,5-tetrahydro-8H-pyrido[4',3':4,5]thieno[3,2-f][1,2,4]triazolo[4m3-a][1,4]-diazepine were dissolved in 12 ml of N,N-dimethylformamide, to which 160 mg of 1,3-dicyclohexylcarbodiimide was added under ice-cooling conditions, followed by agitation at 4°C for 9 hours and then at room temperature for 1 hour. After concentration, a saturated sodium hydrogencarbonate aqueous solution was added, followed by extraction with chloroform and drying with anhydrous magnesium sulfate. This was removed by filtration and the resultant filtrate was concentrated, followed by the resultant residue to silica gel column chromatography (developing solvent: solvent:dichloromethane = 1:99) to obtain 240 mg of the intended compound (yield 80%).

- $^1$H-NMR($CDCl_3$) δ :
  0.4 - 1.1(m,4H), 1.35 - 2.0(m,2H), 2.0 - 2.6(m,1H), 2.09(d,J=6.8Hz,3H), 2.65(s,3H), 2.8 - 4.1(m,2H), 4.1 - 5.3(m,2H), 4.26(q,J=6.8Hz,1H), 6.15(d,J=19.8Hz,IH), 6.31(dd,J=19.8Hz,15.1Hz,1H), 7.1 - 7.55(m,4H)
- MS m/z(Pos. FAB): 478($(M+H)^+$)

Example 12

<u>6-(2-Chlorophenyl)-3-cyclobutanecarbonyl-8,11-dimethyl-2,3,4,5-tetrahydro-8H-pyrido[4',3':4,5] thieno[3,2-f][1,2,4] triazolo[4,3-a][1,4]diazepine</u>

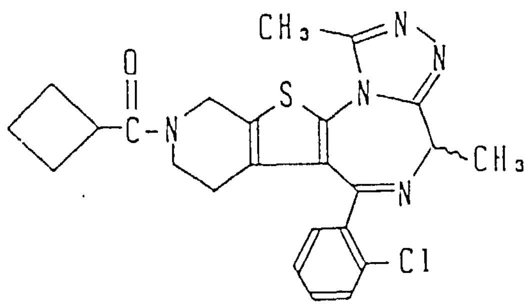

40 mg of cyclobutanecarboxylic acid and 120 mg of 6-(2-chlorophenyl)-8,11-dimethyl-2,3,4,5-tetrahydro-8H-pyrid o[4',3':4,5]thieno[3,2-f][1,2,4]triazolo[4,3-a][1,4] diazepine were dissolved in 8 ml of N,N-dimethylformamide, to which 80 mg of 1,3-dicyclohexylcarbodiimide was added under ice-cooling conditions, followed by agitation at 4°C for 9 hours and then at room temperature for 1 hour. After concentration, a saturated sodium hydrogencarbonate aqueous solution was added, followed by extraction with chloroform and drying with anhydrous magnesium sulfate. This was subjected to filtration and concentrated, and the resultant residue was subjected to silica gel column chromatography (developing solvent: methanol:dichloromethane = 1:99) to obtain 120 mg of the intended compound (yield 82%).

- $^1$H-NMR($CDCl_3$) δ :
  1.2 - 2-.8(m,8H), 2.09(d,J=6.8Hz,3H), 2.65(s,3H), 2.85 - 3.8(m,3H), 3.8 - 4.6(m,2H), 4.25(q,J=6.8Hz,1H), 4.8 - 5.3(m,1H), 7.0 - 7.6(m,4H)
- MS m/z(Pos. FAB): 466($(M+H)^+$)

Example 13

6-(2-Chlorophenyl)-3-cyclopentanecarbonyl-8,11-dimethyl-2,3,4,5-tetrahydro-8H-pyrido[4',3':4,5] thieno[3,2-f][1,2,4] triazolo[4,3-a][1,4]diazepine

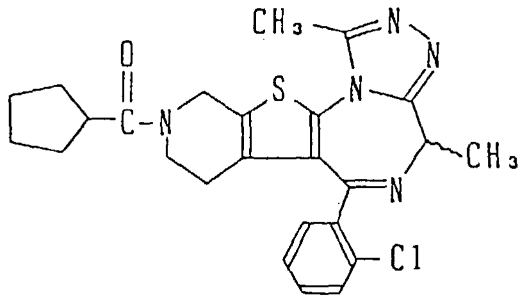

50 mg of cyclopentanecarboxylic acid and 120 mg of 6-(2-chlorophenyl)-8,11-dimethyl-2,3,4,5-tetrahydro-8H-pyrido[4',3':4,5]thieno[3,2-f][1,2,4]triazolo[4,3-a][1,4]-diazepine were dissolved in 8 ml of N,N-dimethylformamide, to which 80 mg of 1,3-dicyclohexylcarbodiimide was added under ice-cooling conditions, followed by agitation at 4°C for 9 hours and then at room temperature for 1 hour. After concentration, a saturated sodium hydrogencarbonate aqueous solution was added, followed by extraction with chloroform and drying with anhydrous magnesium sulfate. This was subjected to filtration and concentrated, and the resultant residue was subjected to silica gel column chromatography (developing solvent: methanol:dichloromethane = 1:99) to obtain 110 mg of the intended compound (yield 73%).

- $^1$H-NMR($CDCl_3$) δ :
  1.1 - 2.1(m,8H), 2.10(d,J=6.8Hz,3H), 2.1 - 3.1(m,1H), 2.66(s,3H), 3.1 - 4.0(m,2H), 4.0 - 5.3(m,2H), 4.26(q, J=6.8Hz,IH), 7.1 - 7.6(m,4H)
- MS m/z(Pos. FAB): 480($(M+H)^+$

## Claims

**Claims for the following Contracting States : AT, BE, CH, DE, FR, GB, IT, LI, LU, NL, SE**

**1.** A triazolo-1,4-di-azepine compound of the below given formula and a pharmacologically acceptable salt thereof,

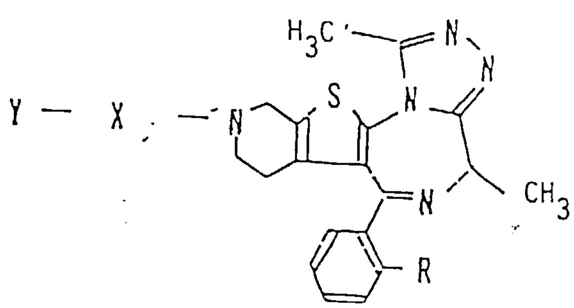

(I)

wherein R represents a hydrogen atom or a halogen atom, X represents a group of the formula

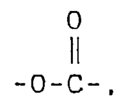

or
a group of the formula

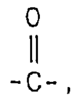

and Y represents

(1) a $C_3$-$C_7$ cycloalkyl group which may have a methyl group as a substituent(s),
(2) a $C_3$-$C_7$ cycloalkyl-$C_1$-$C_6$ alkyl,
(3) an alkynyl group having up to 6 C-atoms,
(4) a group of the formula,

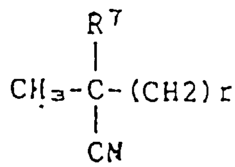

in which R7 is hydrogen or methyl and r is zero, 1 or 2,
(5) a group of the formula, NC-$(CH_2)_P$-, (wherein p is an integer of from 1 to 6).
(6) a group of the formula, A-$(CH_2)_q$- wherein A represents a group selected from a pyridyl group, a pyranyl group and a morpholino group and q is an integer of from 0 to 6),
(7) an alkynyl group having up to 6 C-atoms wherein a phenyl group or a $C_3$-$C_7$ cycloalkyl group is joined to any carbon atom,
(8) a group of the formula,

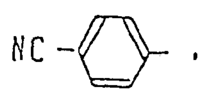

(9) a group of the formula,

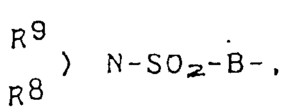

(wherein $R^8$ and $R^9$ are the same or different arid represent a hydrogen atom, a $C_1$-$C_6$ alkyl group, a pyridyl-methyl group or a $C_3$-$C_7$ cycloalkyl group or $R^8$ and $R^9$ may be joined along with a nitrogen atom to form a ring, and B represents a phenylene group or a $C_1$-$C_3$ alkylene group,
(10) a group of the formula,

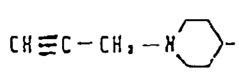

(11) a group of the formula,

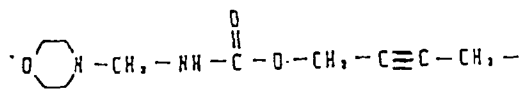

(12) a group of the formula,

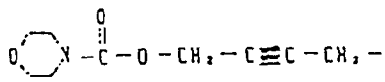

(13) a group of the formula,

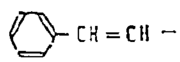

(14) a group of the formula,

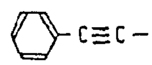

2. The compound and the salt as claimed in Claim 1, in which X is -CO-.

3. The compound and the salt as claimed in Claims 1 or 2 in which Y is a $C_3$-$C_7$ cycloalkyl.

4. The compound and the salt as claimed in Claim 3 in which Y is cyclopropyl.

5. The compound and the salt as claimed in claims 1 or 2 in which Y is HC≡C-C(CH3)2-.

6. The compound and the salt as claimed in Claim 1 in which R is chlorine, and Y-X- is selected from one of the following combinations:

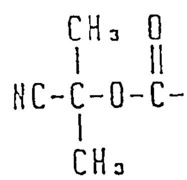

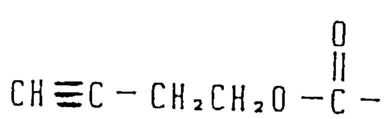

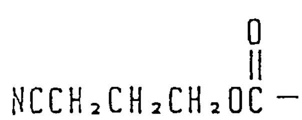

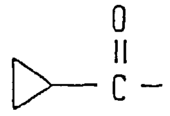

7. The compound and the salt as claimed in claim 1, which is represented by the formula

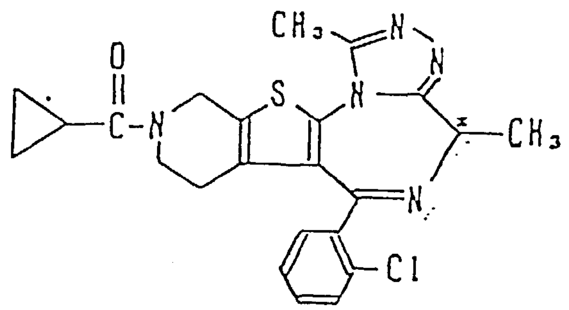

8. The compound and the salt as claimed in claim 7, which is represented by the (+) isomer.

9. A compound represented by the following formula:

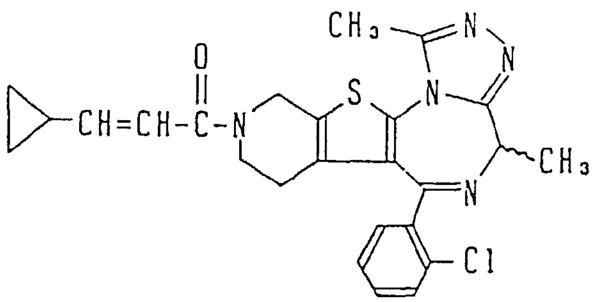

10. A pharmaceutical composition which comprises a pharmacologically effective amount of the compound or the salt thereof, defined in any of the claims 1 to 9 , and a pharmacologically acceptable carrier.

11. The pharmaceutical composition as defined in claim 10, which comprises a pharmacologically effective amount of a compound as defined in claims 3 or 6 or a pharmaceutically acceptable salt thereof.

12. The pharmaceutical composition as defined in claim 10, which comprises a pharmacologically effective amount of the compound as defined in claims 7 or 8.

13. The use of the compound or the salt thereof as defined in any of preceding claims 1 to 9 for making a medicament for anti-PAF activity.

14. The use of the compound or the salt thereof as defined in any of preceding claims 1 to 9 for making a medicament for the treatment or prevention of allergic diseases.

15. The use as claimed in claim 14, in which the disease is asthma.

16. A triazolo-1,4-diazepine derivative represented by the following general formula

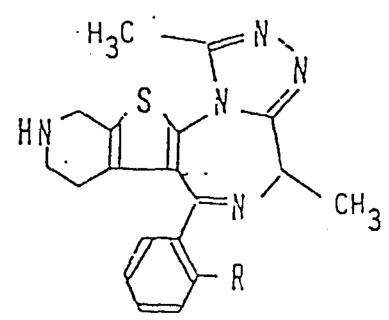

wherein R has the same meaning as defined in claim 1.

**Claims for the following Contracting States : ES, GR**

1. A triazolo-1,4-di-azepine compound of the below given formula and a pharmacologically acceptable salt thereof,

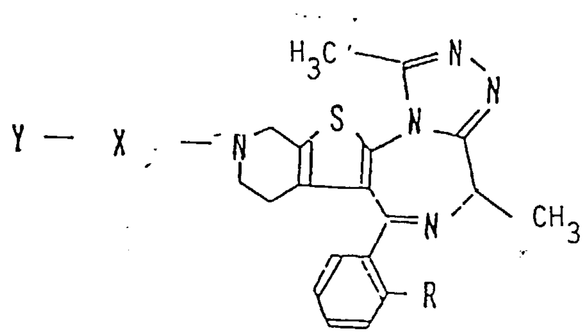

(I)

wherein R represents a hydrogen atom or a halogen atom, X represents a group of the formula

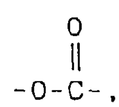

or
a group of the formula.

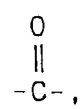

and Y represents

(1) a $C_3$-$C_7$ cycloalkyl group which may have a methyl group as a substituent(s),
(2) a $C_3$-$C_7$ cycloalkyl-$C_1$-$C_6$ alkyl,
(3) an alkynyl group having up to 6 C-atoms,
(4) a group of the formula,

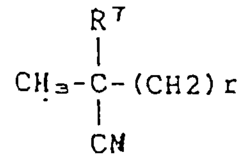

in which R7 is hydrogen or methyl and r is zero, 1 or 2,

(5) a group of the formula, NC-$(CH_2)_P$-, (wherein p is an integer of from 1 to 6).

(6) a group of the formula, A-$(CH_2)_q$- wherein A represents a group selected from a pyridyl group, a pyranyl group and a morpholino group and q is an integer of from 0 to 6),

(7) an alkynyl group having up to 6 C-atoms wherein a phenyl group or a $C_3$-$C_7$ cycloalkyl group is joined to any carbon atom,

(8) a group of the formula,

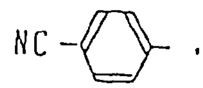

(9) a group of the formula,

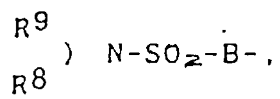

(wherein $R^8$ and $R^9$ are the same or different and represent a hydrogen atom, a $C_1$-$C_6$ alkyl group, a pyridylmethyl group or a $C_3$-$C_7$ cycloalkyl group or $R^8$ and $R^9$ may be joined along with a nitrogen atom to form a ring, and B represents a phenylene group or a $C_1$-$C_3$ alkylene group,

(10) a group of the formula,

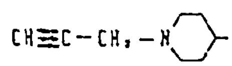

(11) a group of the formula,

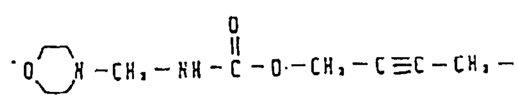

(12) a group of the formula,

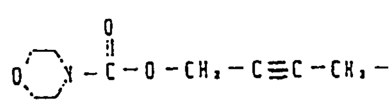

(13) a group of the formula,

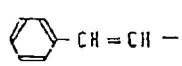

(14) a group of the formula,

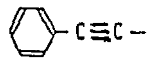

**2.** The compound and the salt as claimed in Claim 1, in which X is -CO-.

**3.** The compound and the salt as claimed in Claims 1 or 2 in which Y is a $C_3$-$C_7$ cycloalkyl.

**4.** The compound and the salt as claimed in Claim 3 in which Y is cyclopropyl.

**5.** The compound and the salt as claimed in Claims 1 or 2 in which Y is HC≡C-C(CH3)2-.

**6.** The compound and the salt as claimed in Claim 1 in which R is chlorine, and Y-X- is selected from one of the following combinations:

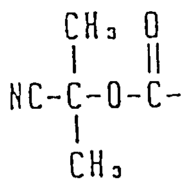

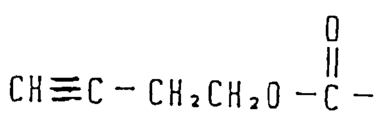

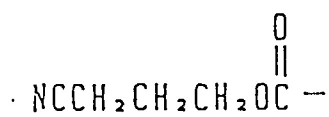

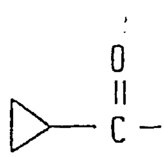

**7.** The compound and the salt as claimed in claim 1, which is represented by the formula

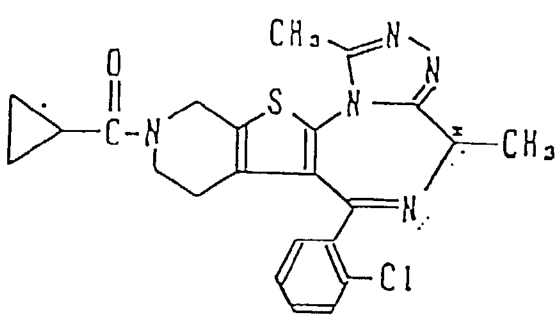

**8.** The compound and the salt as claimed in claim 7, which is represented by the (+) isomer.

**9.** A compound represented by the following formula:

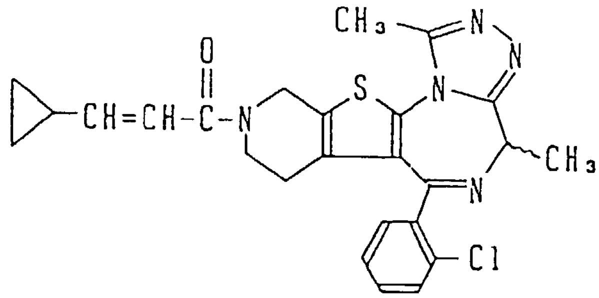

**10.** A pharmaceutical composition which comprises a pharmacologically effective amount of the compound or the salt thereof, defined in any of the claims 1 to 9 , and a pharmacologically acceptable carrier.

**11.** The pharmaceutical composition as defined in claim 10, which comprises a pharmacologically effective amount of a compound as defined in claims 3 or 6 or a pharmaceutically acceptable salt thereof.

**12.** The pharmaceutical composition as defined in claim 10, which comprises a pharmacologically effective amount of the compound as defined in claims 7 or 8.

**13.** The use of the compound or the salt thereof as defined in any of preceding claims 1 to 9 for making a medicament for anti-PAF activity.

**14.** The use of the compound or the salt thereof as defined in any of preceding claims 1 to 9 for making a medicament for the treatment or prevention of allergic diseases.

**15.** The use as claimed in claim 14, in which the disease is asthma.

**16.** A triazolo-1,4-diazepine derivative represented by the following general formula

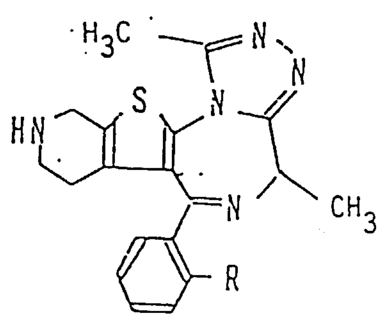

wherein R has the same meaning as defined in claim 1.

**17.** Process for the preparation of a triazolo-1,4-di-azepine compound of the below given formula and a pharmacologically acceptable salt thereof,

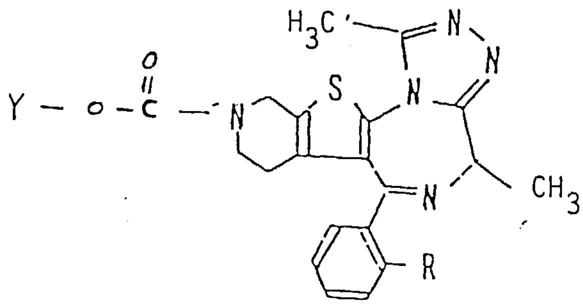

(I')

wherein R represents a hydrogen atom or a halogen atom,
and Y represents

(1) a $C_3$-$C_7$ cycloalkyl group which may have a methyl group as a substituent(s),
(2) a $C_3$-$C_7$ cycloalkyl-$C_1$-$C_6$ alkyl,
(3) an alkynyl group having up to 6 C-atoms,
(4) a group of the formula,

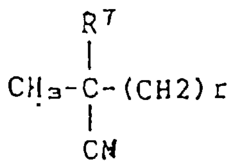

in which R7 is hydgrogen or methyl and r is zero, 1 or 2,
(5) a group of the formula, NC-$(CH_2)_P$-, (wherein p is an integer of from 1 to 6),
(6) a group of the formula, A-$(CH_2)_q$- wherein A represents a group selected from a pyridyl group, a pyranyl group and a morpholino group and q is an integer of from 0 to 6),
(7) an alkynyl group having up to 6 C-atoms wherein a phenyl group or a $C_3$-$C_7$ cycloalkyl group is joined to any carbon atom,
(8) a group of the formula,

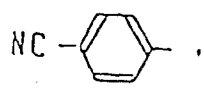

(9) a group of the formula,

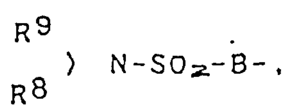

(wherein $R^8$ and $R^9$ are the same or different arid represent a hydrogen atom, a $C_1$-$C_6$ alkyl group, a pyridyl-methyl group or a $C_3$-$C_7$ cycloalkyl group or $R^8$ and $R^9$ may be joined along with a nitrogen atom to form a ring, and B represents a phenylene group or a $C_1$-$C_3$ alkylene group,
(10) a group of the formula,

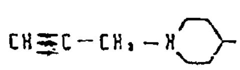

(11) a group of the formula,

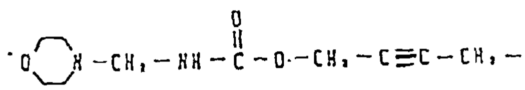

(12) a group of the formula,

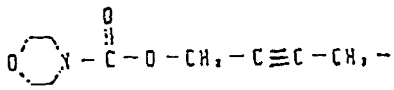

(13) a group of the formula,

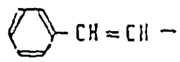

or

(14) a group of the formula,

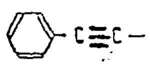 ;

comprising the step of condensing a compound of the general formula (II)

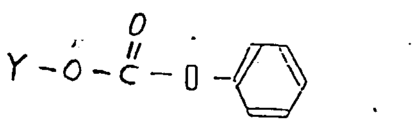 (II)

wherein Y is as defined above
with a compound of the general formula (III)

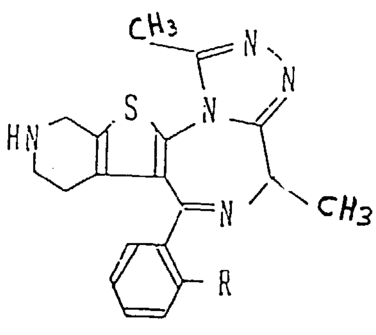 (III)

wherein R is as defined above

**18.** Process for the production of a triazolo-1,4-di-azepine compound of the below given formula and a pharmacologically acceptable salt thereof,

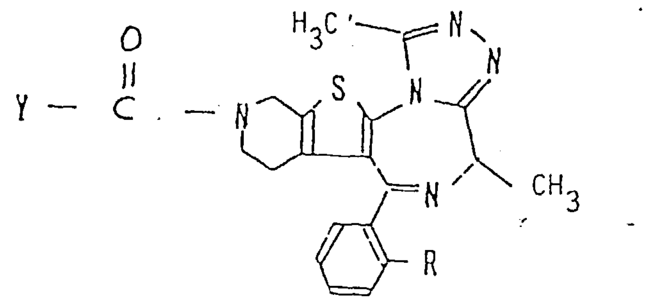

(I'')

wherein R represents a hydrogen atom or a halogen atom,
and Y represents

(1) a $C_3$-$C_7$ cycloalkyl group which may have a methyl group as a substituent(s),
(2) a $C_3$-$C_7$ cycloalkyl-$C_1$-$C_6$ alkyl
(3) an alkynyl group having up to 6 C-atoms,
(4) a group of the formula,

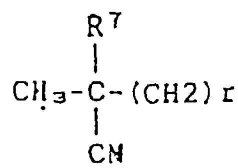

in which R7 is hydgrogen or methyl and r is zero, 1 or 2,
(5) a group of the formula, NC-$(CH_2)_P$-, (wherein p is an integer of from 1 to 6),
(6) a group of the formula, A-$(CH_2)_q$- wherein A represents a group selected from a pyridyl group, a pyranyl group and a morpholino group and q is an integer of from 0 to 6),
(7) an alkynyl group having up to 6 C-atoms wherein a phenyl group or a $C_3$-$C_7$ cycloalkyl group is joined to any carbon atom,
(8) a group of the formula,

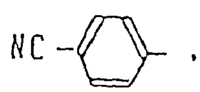

(9) a group of the formula,

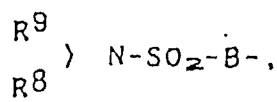

(wherein $R^8$ and $R^9$ are the same or different and represent a hydrogen atom, a $C_1$-$C_6$ alkyl group, a pyridyl-methyl group or a $C_3$-$C_7$ cycloalkyl group or $R^8$ and $R^9$ may be joined along with a nitrogen atom to form a ring, and B represents a phenylene group or a $C_1$-$C_3$ alkylene group,
(10) a group of the formula,

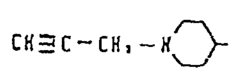

(11) a group of the formula,

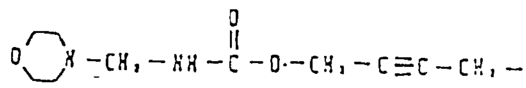

(12) a group of the formula,

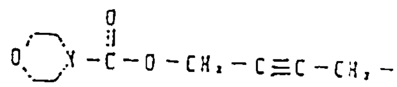

(13) a group of the formula,

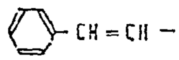

or

(14) a group of the formula,

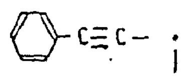

comprising the step of condensing a compound of formula (VI)

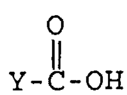

(VI)

wherein Y is as defined above
or its reactive acid derivative with a compound of the formula (III)

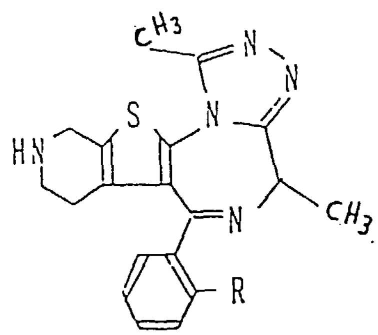

(III)

wherein R is as defined above

**19.** Process for the preparation of

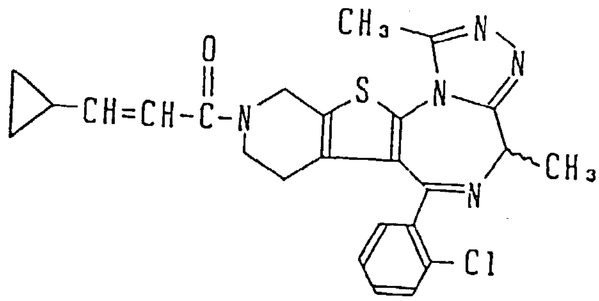

comprising the step of condensing a compound of formula (VI)'

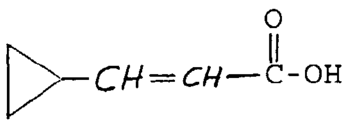

(VI)'

or its reactive acid derivative with a compound of the formula (III)'

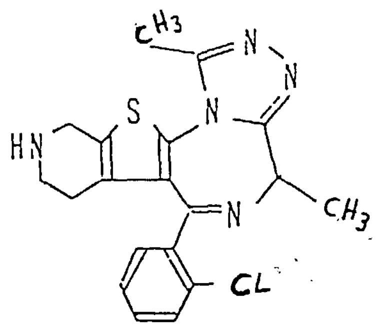

(III)'

## Patentansprüche

### Patentansprüche für folgende Vertragsstaaten : AT, BE, CH, DE, FR, GB, IT, LI, LU, NL, SE

1. Triazolo-1,4-diazepin-Verbindung mit der nachstehend angegebenen Formel und deren pharmakologisch akzeptable Salze

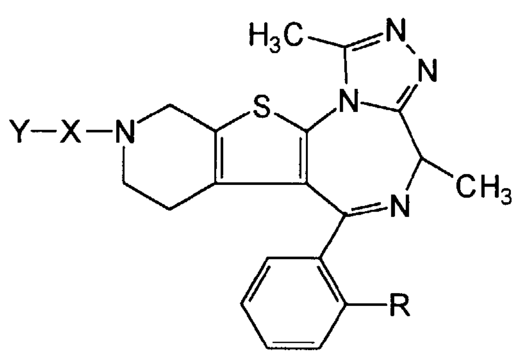

(I)

worin R ein Wasserstoffatom oder ein Halogenatom bedeutet, X eine Gruppe der Formel

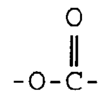

oder eine Gruppe der Formel

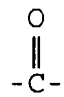

bedeutet, und Y eine

(1) $C_{3-7}$ Cycloalkylgruppe, die eine Methylgruppe als Substituent(en) haben kann,

(2) $C_{3-7}$-Cycloalkyl-$C_{1-6}$-alkyl,

(3) eine Alkinylgruppe mit bis zu 6 Kohlenstoffatomen,

(4) eine Gruppe mit der Formel

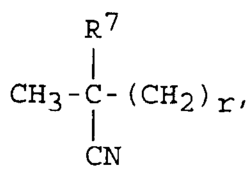

worin $R^7$ Wasserstoff oder Methyl ist und r 0, 1 oder 2 ist,

(5) eine Gruppe mit der Formel NC-$(CH_2)_p$- (worin p eine ganze Zahl von 1 bis 6 darstellt),

(6) eine Gruppe der Formel A-$(CH_2)_q$-, worin A eine Gruppe, ausgewählt aus einer Pyridylgruppe, einer Pyranylgruppe und einer Morpholinogruppe, darstellt und q eine ganze Zahl von 0 bis 6 ist,

(7) eine Alkinylgruppe mit bis zu 6 C-Atomen, worin eine Phenylgruppe oder eine $C_{3-7}$-Cycloalkylgruppe an eines der Kohlenstoffatome gebunden ist,

(8) eine Gruppe mit der Formel

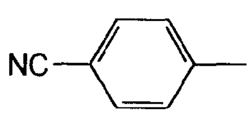

(9) eine Gruppe der Formel

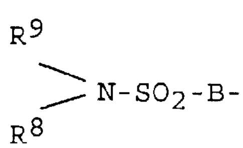

(worin $R^8$ und $R^9$ gleich oder verschieden sind und ein Wasserstoffatom, eine $C_{1-6}$-Alkylgruppe, eine Pyridylmethylgruppe oder eine $C_{3-7}$-Cycloalkylgruppe bedeuten oder $R^8$ und $R^9$ zusammen mit einem Stickstoffatom unter Bildung eines Ringes verbunden sein können, und B eine Phenylengruppe oder eine $C_{1-3}$-Alkylengruppe darstellt,

(10) eine Gruppe der Formel

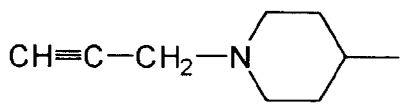

(11) eine Gruppe der Formel

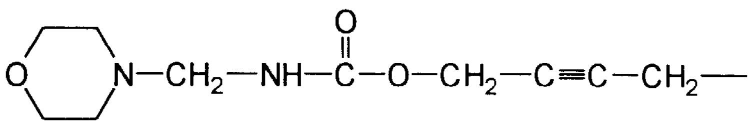

(12) eine Gruppe der Formel

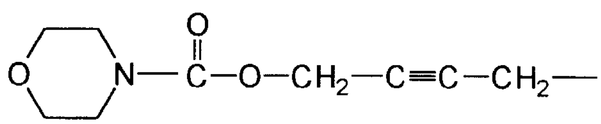

(13) eine Gruppe der Formel

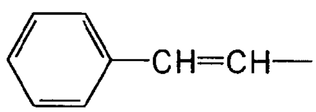

(14) eine Gruppe der Formel

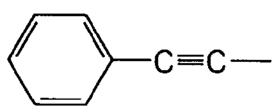

darstellt.

2. Verbindung und Salz gemäss Anspruch 1, worin X -CO- ist.

3. Verbindung und Salz gemäss Anspruch 1 oder 2, worin Y eine $C_{3-7}$-Cycloalkylgruppe ist.

4. Verbindung und Salz gemäss Anspruch 3, worin Y Cyclopropyl ist.

5. Verbindung und Salz gemäss Anspruch 1 oder 2, worin Y $HC{\equiv}C\text{-}C(CH_3)_2$- ist.

6. Verbindung und Salz gemäss Anspruch 1, worin R Chlor ist, und Y-X- aus einer der folgenden Kombinationen ausgewählt wird:

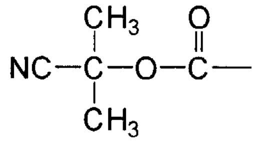

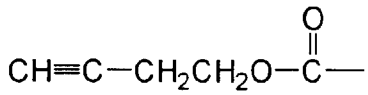

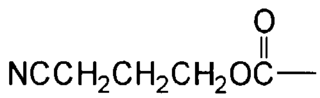

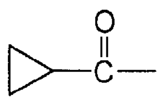

**7.** Verbindung und Salz gemäss Anspruch 1, dargestellt durch die Formel:

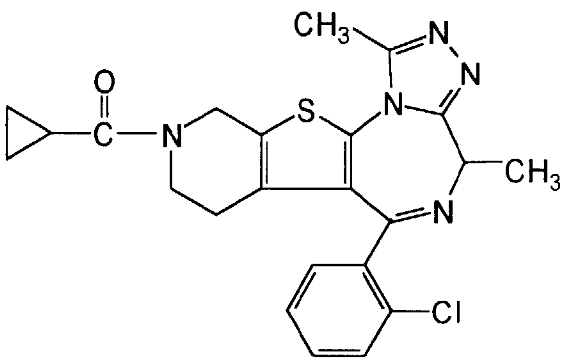

**8.** Verbindung und Salz gemäss Anspruch 7 als (+)-Isomer.

**9.** Verbindung mit der folgenden Formel:

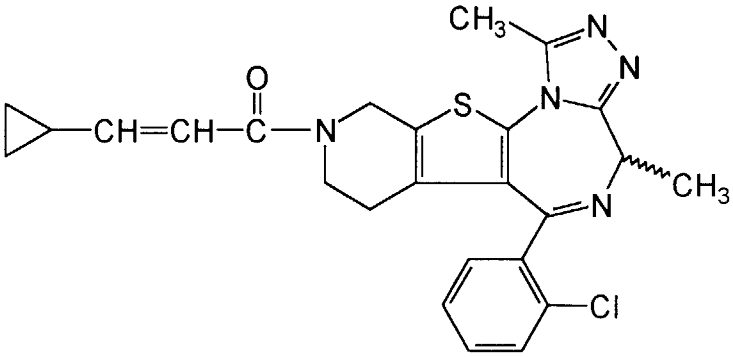

**10.** Pharmazeutische Zusammensetzung, umfassend eine pharmakologisch wirksame Menge der in einem der Ansprüche 1 bis 9 definierten Verbindung bzw. einem Salz davon und einen pharmakologisch akzeptablen Träger.

**11.** Pharmazeutische Zusammensetzung gemäss Anspruch 10, umfassend eine pharmakologisch wirksame Menge einer Verbindung gemäss Anspruch 3 oder 6 oder ein pharmazeutisch akzeptables Salz davon.

**12.** Pharmazeutische Zusammensetzung gemäss Anspruch 10, umfassend eine pharmakologisch wirksame Menge der Verbindung gemäss Anspruch 7 oder 8.

**13.** Verwendung der Verbindung gemäss einem der vorhergehenden Ansprüche 1 bis 9 oder eines Salzes davon zur Herstellung eines Arzneimittels mit Anti-PAF-Aktivität.

**14.** Verwendung der Verbindung gemäss einem der vorhergehenden Ansprüche 1 bis 9 oder eines Salzes davon zur Herstellung eines Medikaments zur Behandlung oder Vorbeugung von allergischen Krankheiten.

**15.** Verwendung gemäss Anspruch 14, wobei die Krankheit Asthma ist.

**16.** Triazolo-1,4-diazepinderivat mit der folgenden allgemeinen Formel:

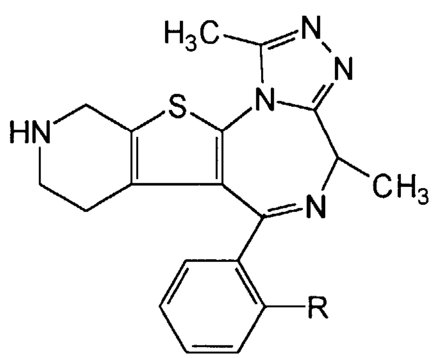

worin R die gleiche Bedeutung wie in Anspruch 1 hat.

**Patentansprüche für folgende Vertragsstaaten : ES, GR**

**1.** Triazolo-1,4-diazepin-Verbindung mit der nachstehend angegebenen Formel und deren pharmakologisch akzeptable Salze

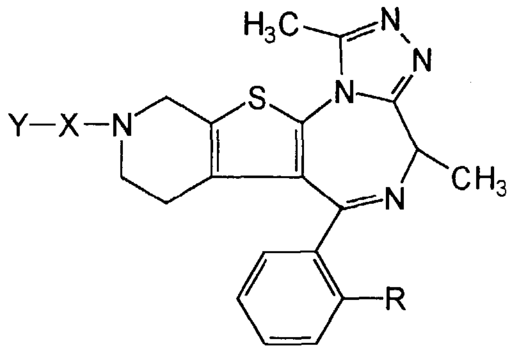

(I)

worin R ein Wasserstoffatom oder ein Halogenatom bedeutet, X eine Gruppe der Formel

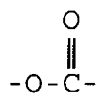

oder eine Gruppe der Formel

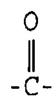

bedeutet, und Y eine

(1) $C_{3-7}$ Cycloalkylgruppe, die eine Methylgruppe als Substituent(en) haben kann,

(2) $C_{3-7}$-Cycloalkyl-$C_{1-6}$-alkyl,

(3) eine Alkinylgruppe mit bis zu 6 Kohlenstoffatomen,

(4) eine Gruppe mit der Formel

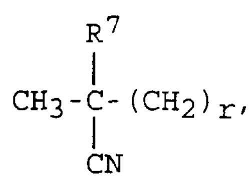

worin $R^7$ Wasserstoff oder Methyl ist und r 0, 1 oder 2 ist,

(5) eine Gruppe mit der Formel $NC-(CH_2)_p-$ (worin p eine ganze Zahl von 1 bis 6 darstellt),

(6) eine Gruppe der Formel $A-(CH_2)_q-$, worin A eine Gruppe, ausgewählt aus einer Pyridylgruppe, einer Pyranylgruppe und einer Morpholinogruppe, darstellt und q eine ganze Zahl von 0 bis 6 ist,

(7) eine Alkinylgruppe mit bis zu 6 C-Atomen, worin eine Phenylgruppe oder eine $C_{3-7}$-Cycloalkylgruppe an eines der Kohlenstoffatome gebunden ist,

(8) eine Gruppe mit der Formel

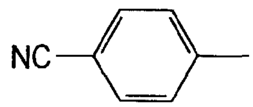

(9) eine Gruppe der Formel

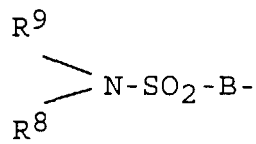

(worin $R^8$ und $R^9$ gleich oder verschieden sind und ein Wasserstoffatom, eine $C_{1-6}$-Alkylgruppe, eine Pyridylmethylgruppe oder eine $C_{3-7}$-Cycloalkylgruppe bedeuten oder $R^8$ und $R^9$ zusammen mit einem Stickstoffatom unter Bildung eines Ringes verbunden sein können, und B eine Phenylengruppe oder eine $C_{1-3}$-Alkylengruppe darstellt,

(10) eine Gruppe der Formel

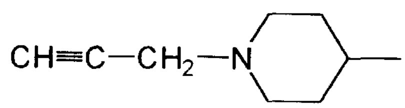

(11) eine Gruppe der Formel

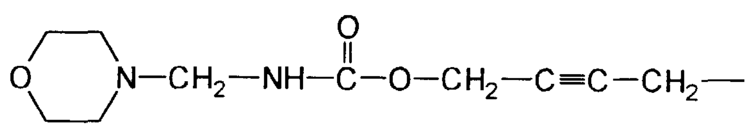

(12) eine Gruppe der Formel

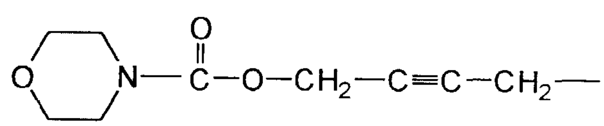

(13) eine Gruppe der Formel

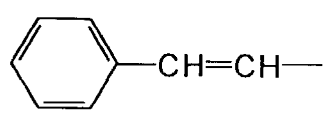

(14) eine Gruppe der Formel

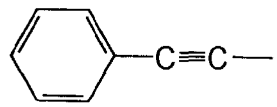

darstellt.

2. Verbindung und Salz gemäss Anspruch 1, worin X -CO- ist.

3. Verbindung und Salz gemäss Anspruch 1 oder 2, worin Y eine $C_{3-7}$-Cycloalkylgruppe ist.

4. Verbindung und Salz gemäss Anspruch 3, worin Y Cyclopropyl ist.

5. Verbindung und Salz gemäss Anspruch 1 oder 2, worin Y $HC{\equiv}C\text{-}C(CH_3)_2\text{-}$ ist.

6. Verbindung und Salz gemäss Anspruch 1, worin R Chlor ist, und Y-X- aus einer der folgenden Kombinationen ausgewählt wird:

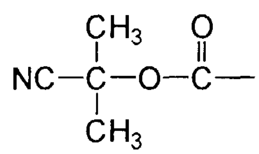

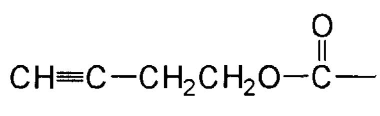

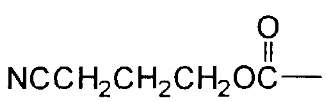

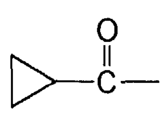

7. Verbindung und Salz gemäss Anspruch 1, dargestellt durch die Formel:

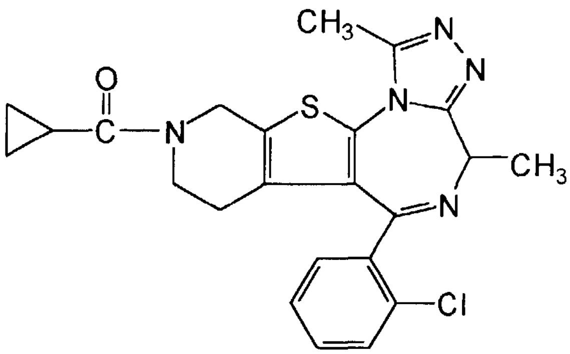

8. Verbindung und Salz gemäss Anspruch 7 als (+)-Isomer.

9. Verbindung mit der folgenden Formel:

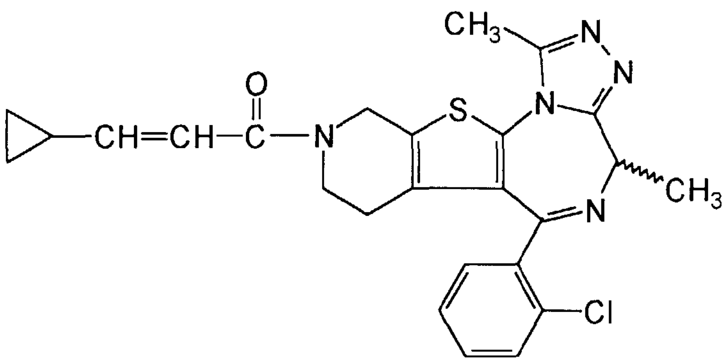

10. Pharmazeutische Zusammensetzung, umfassend eine pharmakologisch wirksame Menge der in einem der Ansprüche 1 bis 9 definierten Verbindung bzw. einem Salz davon und einen pharmakologisch akzeptablen Träger.

11. Pharmazeutische Zusammensetzung gemäss Anspruch 10, umfassend eine pharmakologisch wirksame Menge einer Verbindung gemäss Anspruch 3 oder 6 oder ein pharmazeutisch akzeptables Salz davon.

12. Pharmazeutische Zusammensetzung gemäss Anspruch 10, umfassend eine pharmakologisch wirksame Menge der Verbindung gemäss Anspruch 7 oder 8.

13. Verwendung der Verbindung gemäss einem der vorhergehenden Ansprüche 1 bis 9 oder eines Salzes davon zur Herstellung eines Arzneimittels mit Anti-PAF-Aktivität.

14. Verwendung der Verbindung gemäss einem der vorhergehenden Ansprüche 1 bis 9 oder eines Salzes davon zur Herstellung eines Medikaments zur Behandlung oder Vorbeugung von allergischen Krankheiten.

15. Verwendung gemäss Anspruch 14, wobei die Krankheit Asthma ist.

16. Triazolo-1,4-diazepinderivat mit der folgenden allgemeinen Formel:

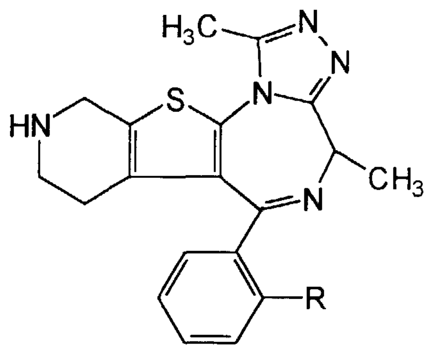

worin R die gleiche Bedeutung wie in Anspruch 1 hat.

**17.** Verfahren zur Herstellung einer Triazolo-1,4-diazepin-Verbindung der nachstehend angegebenen Formel oder eines pharmakologisch akzeptablen Salzes davon

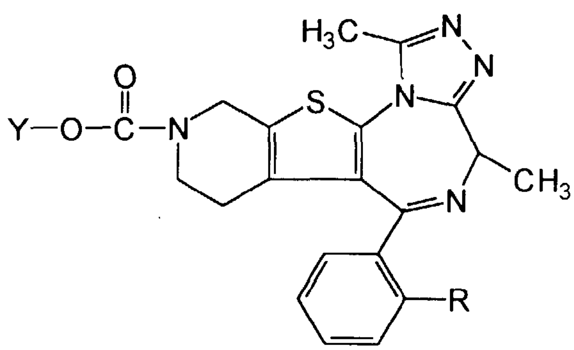

(I')

worin R ein Wasserstoffatom oder ein Halogenatom bedeutet, und Y eine

(1) $C_{3-7}$ Cycloalkylgruppe, die eine Methylgruppe als Substituent(en) haben kann,

(2) $C_{3-7}$-Cycloalkyl-$C_{1-6}$-alkyl,

(3) eine Alkinylgruppe mit bis zu 6 Kohlenstoffatomen,

(4) eine Gruppe mit der Formel

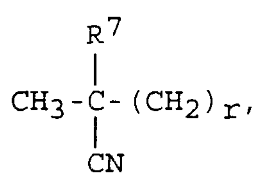

worin $R^7$ Wasserstoff oder Methyl ist und r 0, 1 oder 2 ist,

(5) eine Gruppe mit der Formel NC-$(CH_2)_p$- (worin p eine ganze Zahl von 1 bis 6 darstellt),

(6) eine Gruppe der Formel A-$(CH_2)_q$-, worin A eine Gruppe, ausgewählt aus einer Pyridylgruppe, einer Pyranylgruppe und einer Morpholinogruppe, darstellt und q eine ganze Zahl von 0 bis 6 ist,

(7) eine Alkinylgruppe mit bis zu 6 C-Atomen, worin eine Phenylgruppe oder eine $C_{3-7}$-Cycloalkylgruppe an eines der Kohlenstoffatome gebunden ist,

(8) eine Gruppe mit der Formel

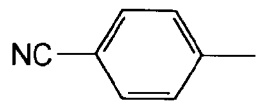

(9) eine Gruppe der Formel

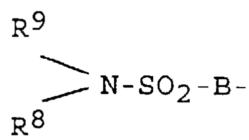

(worin $R^8$ und $R^9$ gleich oder verschieden sind und ein Wasserstoffatom, eine $C_{1-6}$-Alkylgruppe, eine Pyridylmethylgruppe oder eine $C_{3-7}$-Cycloalkylgruppe bedeuten oder $R^8$ und $R^9$ zusammen mit einem Stickstoffatom unter Bildung eines Ringes verbunden sein können, und B eine Phenylengruppe oder eine $C_{1-3}$-Alkylengruppe darstellt,

(10) eine Gruppe der Formel

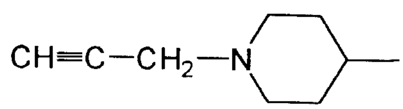

(11) eine Gruppe der Formel

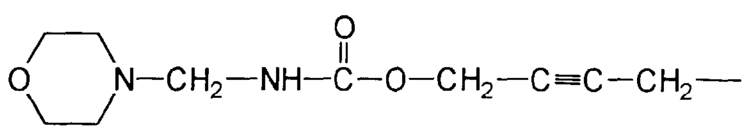

(12) eine Gruppe der Formel

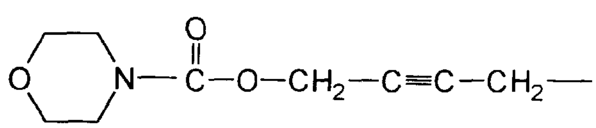

(13) eine Gruppe der Formel

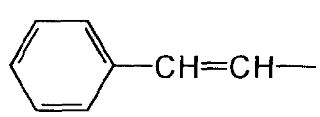

oder

(14) eine Gruppe der Formel

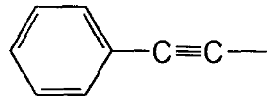

darstellt, umfassend den Schritt der Kondensierung einer Verbindung mit der allgemeinen Formel (II)

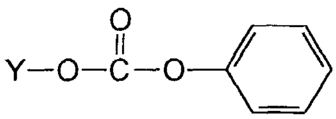

( II )

worin Y wie oben definiert ist, mit einer Verbindung der allgemeinen Formel (III)

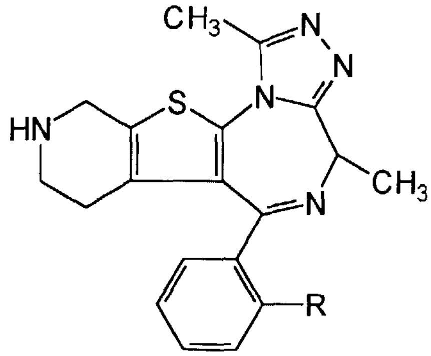

( III )

worin R wie oben definiert ist.

**18.** Verfahren zur Herstellung einer Triazolo-1,4-diazepin-Verbindung mit der nachstehend angegebenen Formel oder eines pharmakologisch akzeptablen Salzes davon:

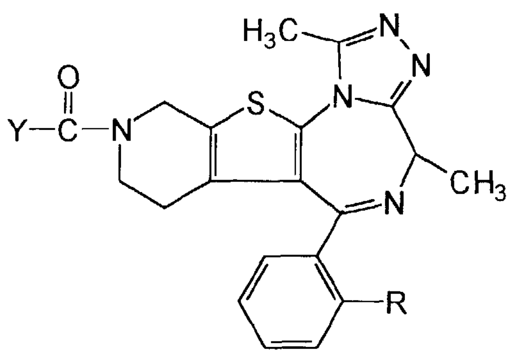

( I" )

worin R ein Wasserstoffatom oder ein Halogenatom bedeutet, und Y eine

(1) $C_{3-7}$ Cycloalkylgruppe, die eine Methylgruppe als Substituent(en) haben kann,

(2) $C_{3-7}$-Cycloalkyl-$C_{1-6}$-alkyl,

(3) eine Alkinylgruppe mit bis zu 6 Kohlenstoffatomen,

(4) eine Gruppe mit der Formel

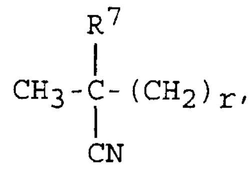

worin $R^7$ Wasserstoff oder Methyl ist und r 0, 1 oder 2 ist,

(5) eine Gruppe mit der Formel $NC-(CH_2)_p-$ (worin p eine ganze Zahl von 1 bis 6 darstellt),

(6) eine Gruppe der Formel $A-(CH_2)_q-$, worin A eine Gruppe, ausgewählt aus einer Pyridylgruppe, einer Pyranylgruppe und einer Morpholinogruppe, darstellt und q eine ganze Zahl von 0 bis 6 ist,

(7) eine Alkinylgruppe mit bis zu 6 C-Atomen, worin eine Phenylgruppe oder eine $C_{3-7}$-Cycloalkylgruppe an eines der Kohlenstoffatome gebunden ist,

(8) eine Gruppe mit der Formel

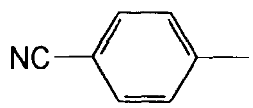

(9) eine Gruppe der Formel

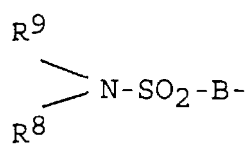

(worin $R^8$ und $R^9$ gleich oder verschieden sind und ein Wasserstoffatom, eine $C_{1-6}$-Alkylgruppe, eine Pyridylmethylgruppe oder eine $C_{3-7}$-Cycloalkylgruppe bedeuten oder $R^8$ und $R^9$ zusammen mit einem Stickstoffatom unter Bildung eines Ringes verbunden sein können, und B eine Phenylengruppe oder eine $C_{1-3}$-Alkylengruppe darstellt,

(10) eine Gruppe der Formel

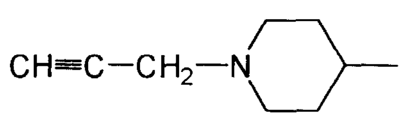

(11) eine Gruppe der Formel

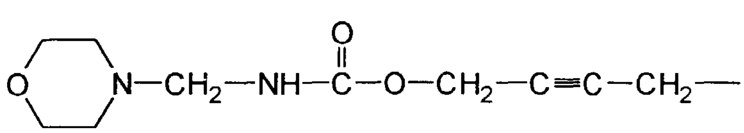

(12) eine Gruppe der Formel

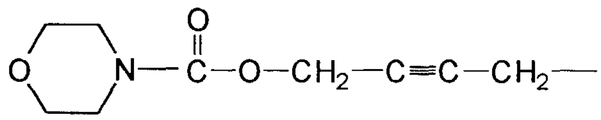

(13) eine Gruppe der Formel

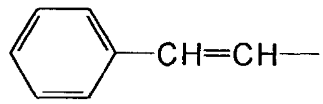

oder

(14) eine Gruppe der Formel

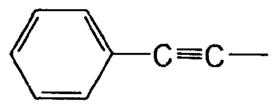

darstellt, umfassend den Schritt der Kondensierung einer Verbindung der Formel (VI)

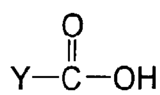

(VI)

worin Y wie oben definiert ist, oder eines reaktiven Säurederivats davon mit einer Verbindung der allgemeinen Formel (III)

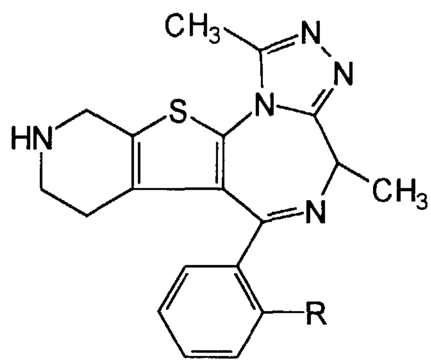

(III)

worin R wie oben definiert ist.

**19.** Verfahren zur Herstellung von

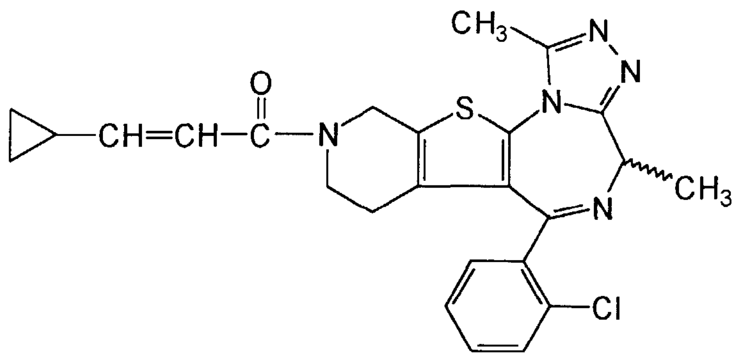

umfassend den Schritt der Kondensierung einer Verbindung der Formel (VI')

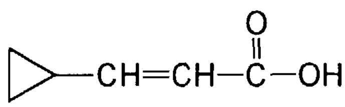

(VI')

oder eines reaktiven Derivats davon mit einer Verbindung der Formel (III')

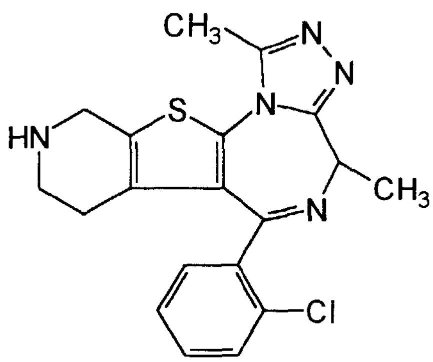

(III')

## Revendications

**Revendications pour les Etats contractants suivants : AT, BE, CH, DE, FR, GB, IT, LI, LU, NL, SE**

**1.** Composé de triazolo-1,4-diazépine répondant à la formule donnée ci-dessous et sel pharmacologiquement acceptable de celui-ci,

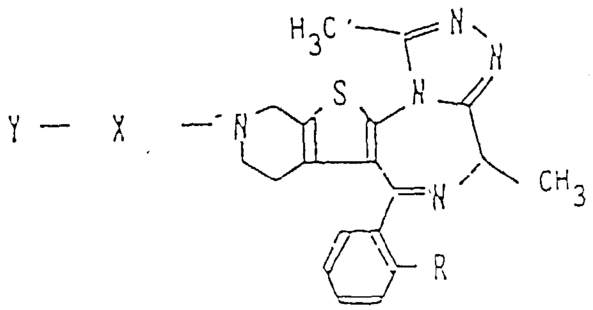

(I)

où R représente un atome d'hydrogène ou un atome d'halogène, X représente un groupe de formule

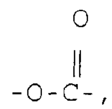

ou
un groupe de formule

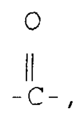

et Y représente

(1) un groupe cycloalkyle en $C_3$ à $C_7$ qui peut porter le groupe méthyle comme substituant(s),
(2) un groupe (cycloalkyl en $C_3$ à $C_7$)(alkyle en $C_1$ à $C_6$),
(3) un groupe alcynyle ayant jusqu'à 6 atomes de carbone,
(4) un groupe de formule

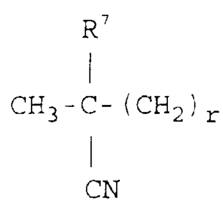

dans laquelle $R^7$ représente un atome d'hydrogène ou un groupe méthyle et r est égal à zéro, 1 ou 2,
(5) un groupe de formule $NC-(CH_2)_p-$ (dans laquelle p représente un nombre entier de 1 à 6),
(6) un groupe de formule $A-(CH_2)_q-$, dans laquelle A représente un groupe choisi parmi un groupe pyridyle, un groupe pyranyle et un groupe morpholino et q représente un nombre entier de 0 à 6,
(7) un groupe alcynyle ayant jusqu'à 6 atomes de carbone dans lequel un groupe phényle ou un groupe cycloalkyle en $C_3$ à $C_7$ est lié à n'importe quel atome de carbone,
(8) un groupe de formule

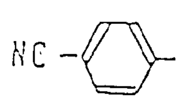

.

(9) un groupe de formule

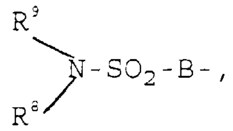

(dans laquelle $R^8$ et $R^9$ sont identiques ou différents et représentent un atome d'hydrogène, un groupe alkyle en $C_1$ à $C_6$, un groupe pyridylméthyle ou un groupe cycloalkyle en $C_3$ à $C_7$ ou $R^8$ et $R^9$ peuvent être liés avec un atome d'azote pour former un cycle, et B représente un groupe phénylène ou un groupe alkylène en $C_1$ à $C_3$,
(10) un groupe de formule,

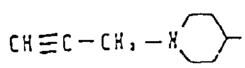

(11) un groupe de formule,

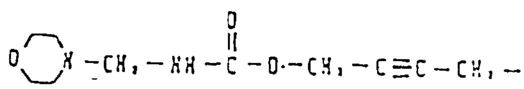

(12) un groupe de formule,

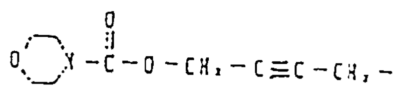

(13) un groupe de formule,

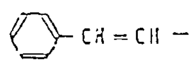

(14) un groupe de formule,

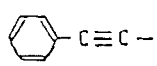

**2.** Composé et sel selon la revendication 1, dans lesquels X représente -CO-.

**3.** Composé et sel selon la revendication 1 ou 2, dans lesquels Y représente un groupe cycloalkyle en $C_3$ à $C_7$.

**4.** Composé et sel selon la revendication 3, dans lesquels Y représente un groupe cyclopropyle.

**5.** Composé et sel selon la revendication 1 ou 2, dans lesquels Y représente $HC{\equiv}C\text{-}C(CH_3)_2\text{-}$.

**6.** Composé et sel selon la revendication 1, dans lesquels R représente un atome de chlore et Y-X- est choisi parmi les combinaisons suivantes :

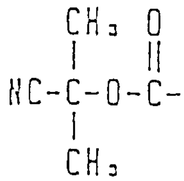

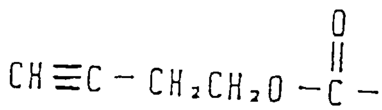

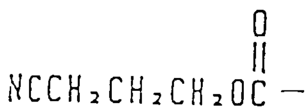

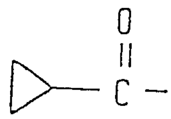

**7.** Composé et sel selon la revendication 1, qui sont représentés par la formule :

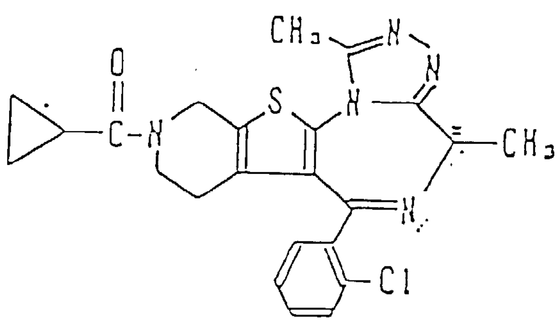

**8.** Composé et sel selon la revendication 7, qui sont représentés par l'isomère (+).

**9.** Composé représenté par la formule suivante :

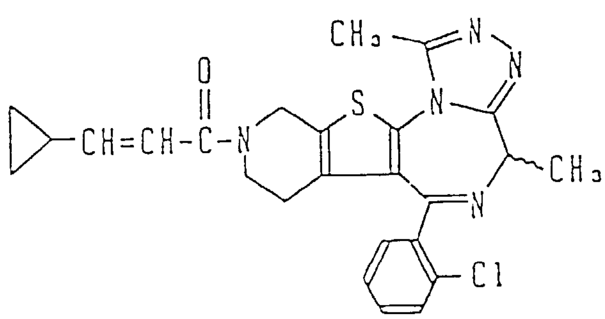

**10.** Composition pharmaceutique qui comprend une quantité pharmacologiquement efficace du composé ou du sel de celui-ci, selon l'une quelconque des revendications 1 à 9, et un véhicule pharmacologiquement acceptable.

**11.** Composition pharmaceutique selon la revendication 10, qui comprend une quantité pharmacologiquement efficace d'un composé selon la revendication 3 ou 6 ou d'un sel pharmaceutiquement acceptable de celui-ci.

**12.** Composition pharmaceutique selon la revendication 10, qui comprend une quantité pharmacologiquement efficace du composé selon la revendication 7 ou 8.

**13.** Utilisation du composé ou du sel de celui-ci selon l'une quelconque des revendications précédentes 1 à 9 pour fabriquer un médicament doté d'une activité anti-PAF (Facteur d'Activation des Plaquettes).

**14.** Utilisation du composé ou du sel de celui-ci selon l'une quelconque des revendications précédentes 1 à 9 pour la fabrication d'un médicament pour le traitement ou la prévention des maladies allergiques.

**15.** Utilisation selon la revendication 14, dans laquelle la maladie est l'asthme.

**16.** Dérivé de triazolo-1,4-diazépine représenté par la formule générale suivante :

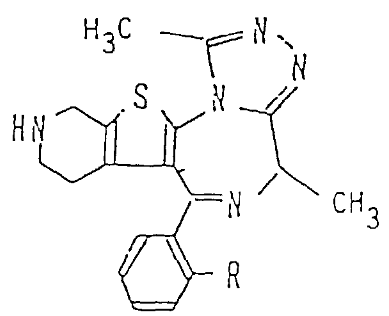

dans laquelle R a la même signification que dans la revendication 1.

**Revendications pour les Etats contractants suivants : ES, GR**

**1.** Composé de triazolo-1,4-diazépine répondant à la formule donnée ci-dessous et sel pharmacologiquement acceptable de celui-ci,

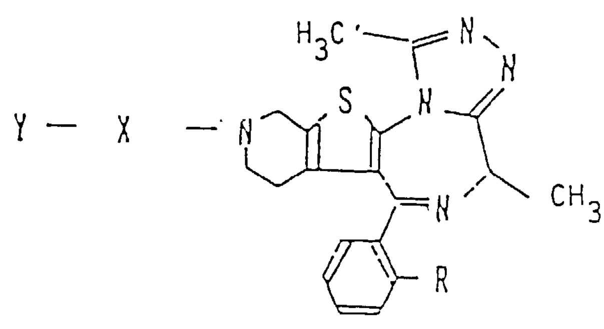

(I)

où R représente un atome d'hydrogène ou un atome d'halogène, X représente un groupe de formule

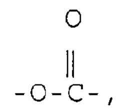

ou
un groupe de formule

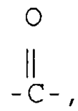

et Y représente

(1) un groupe cycloalkyle en $C_3$ à $C_7$ qui peut porter le groupe méthyle comme substituant(s),
(2) un groupe (cycloalkyl en $C_3$ à $C_7$)(alkyle en $C_1$ à $C_6$),
(3) un groupe alcynyle ayant jusqu'à 6 atomes de carbone,
(4) un groupe de formule

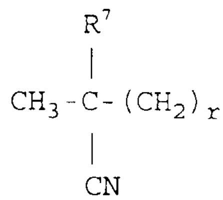

dans laquelle $R^7$ représente un atome d'hydrogène ou un groupe méthyle et r est égal à zéro, 1 ou 2,
(5) un groupe de formule $NC-(CH_2)_p-$ (dans laquelle p représente un nombre entier de 1 à 6),
(6) un groupe de formule $A-(CH_2)_q-$, dans laquelle A représente un groupe choisi parmi un groupe pyridyle, un groupe pyranyle et un groupe morpholino et q représente un nombre entier de 0 à 6,
(7) un groupe alcynyle ayant jusqu'à 6 atomes de carbone dans lequel un groupe phényle ou un groupe cycloalkyle en $C_3$ à $C_7$ est lié à n'importe quel atome de carbone,
(8) un groupe de formule

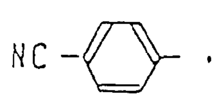

(9) un groupe de formule

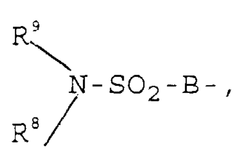

(dans laquelle $R^8$ et $R^9$ sont identiques ou différents et représentent un atome d'hydrogène, un groupe alkyle en $C_1$ à $C_6$, un groupe pyridylméthyle ou un groupe cycloalkyle en $C_3$ à $C_7$ ou $R^8$ et $R^9$ peuvent être liés avec un atome d'azote pour former un cycle, et B représente un groupe phénylène ou un groupe alkylène en $C_1$ à $C_3$,
(10) un groupe de formule,

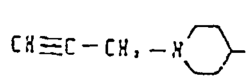

(11) un groupe de formule,

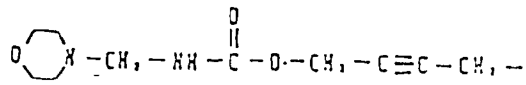

(12) un groupe de formule,

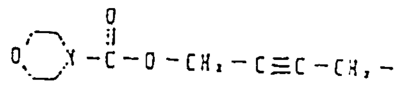

(13) un groupe de formule,

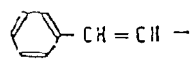

(14) un groupe de formule,

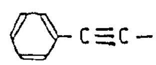

**2.** Composé et sel selon la revendication 1, dans lesquels X représente -CO-.

**3.** Composé et sel selon la revendication 1 ou 2, dans lesquels Y représente un groupe cycloalkyle en $C_3$ à $C_7$.

**4.** Composé et sel selon la revendication 3, dans lesquels Y représente un groupe cyclopropyle.

**5.** Composé et sel selon la revendication 1 ou 2, dans lesquels Y représente $HC{\equiv}C\text{-}C(CH_3)_2\text{-}$.

**6.** Composé et sel selon la revendication 1, dans lesquels R représente un atome de chlore et Y-X- est choisi parmi les combinaisons suivantes :

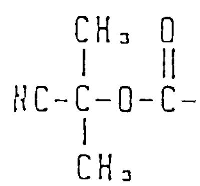

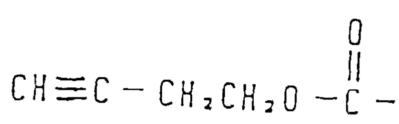

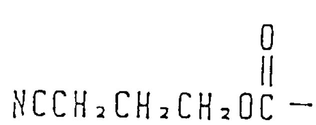

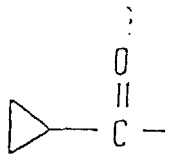

7. Composé et sel selon la revendication 1, qui sont représentés par la formule :

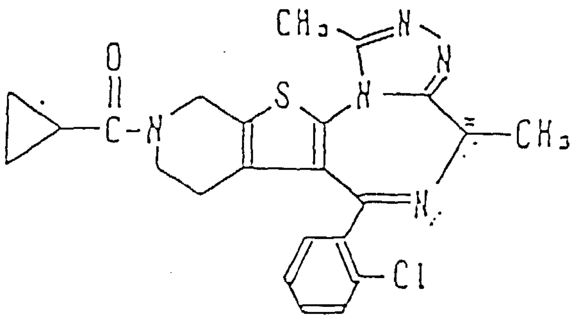

8. Composé et sel selon la revendication 7, qui sont représentés par l'isomère (+).

9. Composé représenté par la formule suivante :

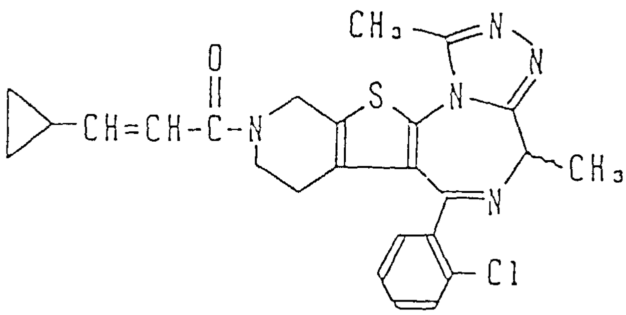

10. Composition pharmaceutique qui comprend une quantité pharmacologiquement efficace du composé ou du sel de celui-ci, selon l'une quelconque des revendications 1 à 9, et un véhicule pharmacologiquement acceptable.

11. Composition pharmaceutique selon la revendication 10, qui comprend une quantité pharmacologiquement efficace d'un composé selon la revendication 3 ou 6 ou d'un sel pharmaceutiquement acceptable de celui-ci.

12. Composition pharmaceutique selon la revendication 10, qui comprend une quantité pharmacologiquement efficace du composé selon la revendication 7 ou 8.

13. Utilisation du composé ou du sel de celui-ci selon l'une quelconque des revendications précédentes 1 à 9 pour fabriquer un médicament doté d'une activité anti-PAF (Facteur d'Activation des Plaquettes).

14. Utilisation du composé ou du sel de celui-ci selon l'une quelconque des revendications précédentes 1 à 9 pour la fabrication d'un médicament pour le traitement ou la prévention des maladies allergiques.

15. Utilisation selon la revendication 14, dans laquelle la maladie est l'asthme.

**16.** Dérivé de triazolo-1,4-diazépine représenté par la formule générale suivante :

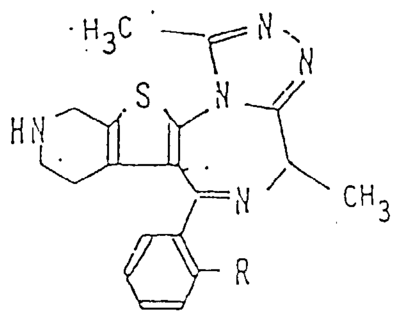

dans laquelle R a la même signification que dans la revendication 1.

**17.** Procédé de préparation d'un composé de triazolo-1,4-diazépine répondant à la formule donnée ci-dessous et d'un sel pharmacologiquement acceptable de celui-ci,

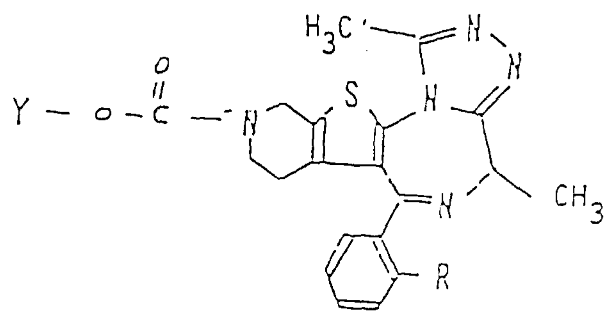

(I')

où R représente un atome d'hydrogène ou un atome d'halogène,
et Y représente

(1) un groupe cycloalkyle en $C_3$ à $C_7$ qui peut porter le groupe méthyle comme substituant(s),
(2) un groupe (cycloalkyl en $C_3$ à $C_7$)(alkyle en $C_1$ à $C_6$),
(3) un groupe alcynyle ayant jusqu'à 6 atomes de carbone,
(4) un groupe de formule

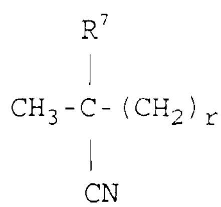

dans laquelle $R^7$ représente un atome d'hydrogène ou un groupe méthyle et r est égal à zéro, 1 ou 2,
(5) un groupe de formule $NC-(CH_2)_p-$ (dans laquelle p représente un nombre entier de 1 à 6),
(6) un groupe de formule $A-(CH_2)_q-$, dans laquelle A représente un groupe choisi parmi un groupe pyridyle, un groupe pyranyle et un groupe morpholino et q représente un nombre entier de 0 à 6,
(7) un groupe alcynyle ayant jusqu'à 6 atomes de carbone dans lequel un groupe phényle ou un groupe cycloalkyle en $C_3$ à $C_7$ est lié à n'importe quel atome de carbone,
(8) un groupe de formule

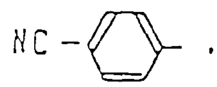

(9) un groupe de formule

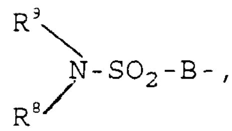

(dans laquelle $R^8$ et $R^9$ sont identiques ou différents et représentent un atome d'hydrogène, un groupe alkyle en $C_1$ à $C_6$, un groupe pyridylméthyle ou un groupe cycloalkyle en $C_3$ à $C_7$ ou $R^8$ et $R^9$ peuvent être liés avec un atome d'azote pour former un cycle, et B représente un groupe phénylène ou un groupe alkylène en $C_1$ à $C_3$,
(10) un groupe de formule,

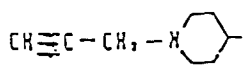

(11) un groupe de formule,

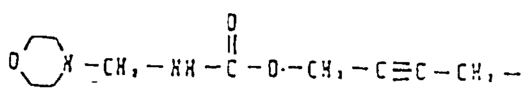

(12) un groupe de formule,

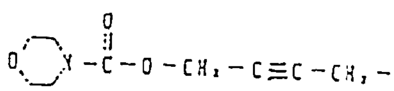

(13) un groupe de formule,

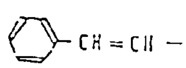

or
(14) un groupe de formule,

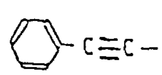

comprenant l'étape consistant à condenser un composé de formule générale (II)

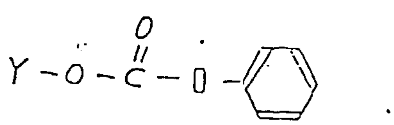

(II)

dans laquelle Y est tel que défini ci-dessus avec un composé de formule générale (III)

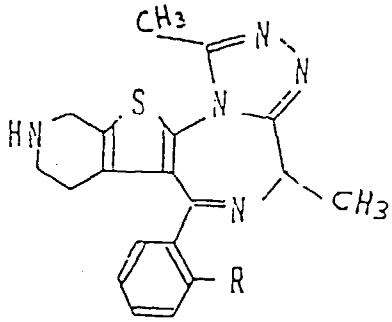

(III)

dans laquelle R est tel que défini ci-dessus.

**18.** Procédé de production d'un composé de triazolo-1,4-diazépine répondant à la formule donnée ci-dessous et d'un sel pharmacologiquement acceptable de celui-ci,

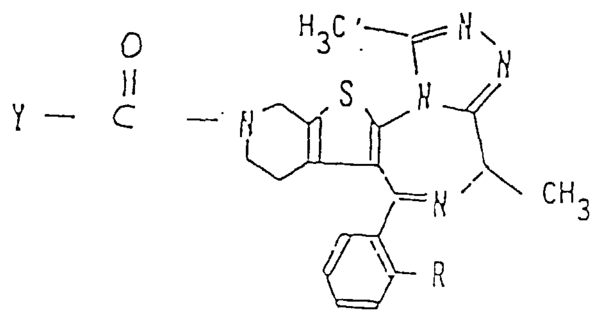

(I'')

où R représente un atome d'hydrogène ou un atome d'halogène,
et Y représente

(1) un groupe cycloalkyle en $C_3$ à $C_7$ qui peut porter le groupe méthyle comme substituant(s),
(2) un groupe (cycloalkyl en $C_3$ à $C_7$)(alkyle en $C_1$ à $C_6$),
(3) un groupe alcynyle ayant jusqu'à 6 atomes de carbone,
(4) un groupe de formule

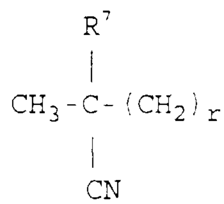

dans laquelle $R^7$ représente un atome d'hydrogène ou un groupe méthyle et r est égal à zéro, 1 ou 2,
(5) un groupe de formule $NC-(CH_2)_p-$ (dans laquelle p représente un nombre entier de 1 à 6),
(6) un groupe de formule $A-(CH_2)_q-$, dans laquelle A représente un groupe choisi parmi un groupe pyridyle, un groupe pyranyle et un groupe morpholino et q représente un nombre entier de 0 à 6,
(7) un groupe alcynyle ayant jusqu'à 6 atomes de carbone dans lequel un groupe phényle ou un groupe cycloalkyle en $C_3$ à $C_7$ est lié à n'importe quel atome de carbone,
(8) un groupe de formule

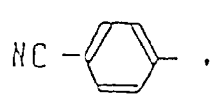

(9) un groupe de formule

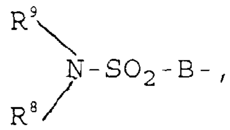

(dans laquelle $R^8$ et $R^9$ sont identiques ou différents et représentent un atome d'hydrogène, un groupe alkyle en $C_1$ à $C_6$, un groupe pyridylméthyle ou un groupe cycloalkyle en $C_3$ à $C_7$ ou $R^8$ et $R^9$ peuvent être liés avec un atome d'azote pour former un cycle, et B représente un groupe phénylène ou un groupe alkylène en $C_1$ à $C_3$,
(10) un groupe de formule,

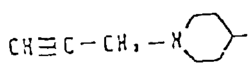

(11) un groupe de formule,

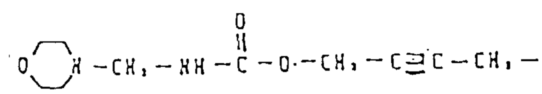

(12) un groupe de formule,

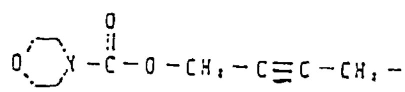

(13) un groupe de formule,

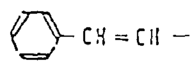

or
(14) un groupe de formule,

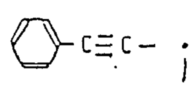

comprenant l'étape consistant à condenser un composé de formule générale (VI)

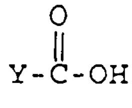

(VI)

dans laquelle Y est tel que défini ci-dessus
ou son dérivé d'acide réactif avec un composé de formule (III)

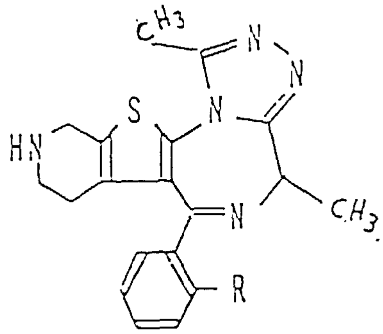

( III )

dans laquelle R est tel que défini ci-dessus.

**19.** Procédé de préparation de

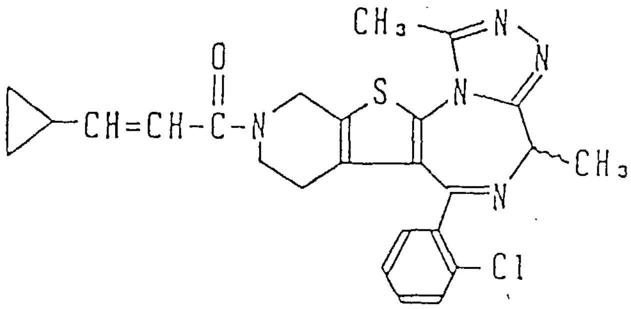

comprenant l'étape consistant à condenser un composé de formule (VI)'

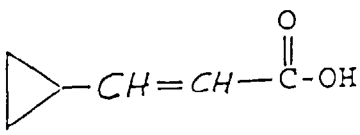

(VI)'

ou son dérivé d'acide réactif avec un composé de formule (III)'

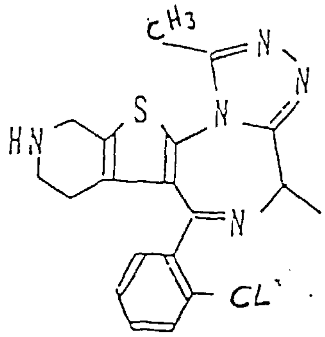

(III)'